(19) Europäisches Patentamt
European Patent Office
Office européen des brevets

(11) EP 2 428 235 B1

(12) **EUROPÄISCHE PATENTSCHRIFT**

(45) Veröffentlichungstag und Bekanntmachung des Hinweises auf die Patenterteilung:
**04.06.2014 Patentblatt 2014/23**

(51) Int Cl.:
***A61L 27/56*** *(2006.01)* ***C08F 18/14*** *(2006.01)*

(21) Anmeldenummer: **11005069.7**

(22) Anmeldetag: **21.11.2008**

(54) **Verwendung von durch Polymerisation härtbaren Zusammensetzungen zur Herstellung biologisch abbaubarer, bioverträglicher, vernetzter Polymere**

Use of compounds hardened by means of polymerisation for producing biodegradable, biocompatible, networked polymers

Utilisation de compositions durcissables par polymérisation pour la fabrication de polymères biodégradables, biocompatibles et mis en réseau

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **23.11.2007 AT 19032007**
**25.03.2008 AT 4612008**

(43) Veröffentlichungstag der Anmeldung:
**14.03.2012 Patentblatt 2012/11**

(62) Dokumentnummer(n) der früheren Anmeldung(en) nach Art. 76 EPÜ:
**08852256.0 / 2 219 697**

(73) Patentinhaber: **Technische Universität Wien**
**1040 Wien (AT)**

(72) Erfinder:
- **Liska, Robert**
  **2123 Schleinbach (AT)**
- **Stampfl, Jürgen**
  **1040 Wien (AT)**
- **Varga, Franz**
  **3001 Mauerbach (AT)**
- **Gruber, Heinrich**
  **1190 Wien (AT)**
- **Baudis, Stefan**
  **1120 Wien (AT)**
- **Heller, Christian**
  **1040 Wien (AT)**
- **Schuster, Monika**
  **1050 Wien (AT)**
- **Bergmeister, Helga**
  **1030 Wien (AT)**
- **Weigel, Günter**
  **1160 Wien (AT)**
- **Dworak, Claudia**
  **1230 Wien (AT)**

(74) Vertreter: **Ellmeyer, Wolfgang**
**Patentanwalt**
**Mariahilferstrasse 50**
**1070 Wien (AT)**

(56) Entgegenhaltungen:
**EP-A1- 1 245 636** **WO-A2-2004/087777**
**US-B1- 6 979 460**

- **LINO FERREIRA, MARIA H. GIL, ANTONIO M.S. CABRITA, JONATHAN S. DORDICK: "Biocatalytic synthesis of highly ordered degradable dextran-based hydrogels", BIOMATERIALS, Bd. 26, Nr. 23, August 2005 (2005-08), Seiten 4707-4716, XP002667149,**
- **LIU, YUAN-GANG ET AL: "Synthesis and application of biodegradable crosslinking agent", HUAQIAO DAXUE XUEBAO, ZIRAN KEXUEBAN , 28(4), 394-398 CODEN: HDZIEF; ISSN: 1000-5013, Oktober 2007 (2007-10), XP009158590,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 428 235 B1

## Beschreibung

Die vorliegende Erfindung betrifft die Verwendung von durch Polymerisation härtbaren Zusammensetzungen zur Herstellung biologisch abbaubarer, bioverträglicher, vernetzter Polymere, vorzugsweise auf Basis von Polyvinylalkohol.

STAND DER TECHNIK

Auf dem Gebiet der medizinischen Chemie wurden seit vielen Jahren Anstrengungen unternommen, biologisch abbaubare Kunststoffe und daraus hergestellte Formkörper als Implantate für den menschlichen und tierischen Körper zu entwickeln, die beispielsweise als Stütz- und Aufbaumaterial für Gewebe (z.B. Knochen), dienen können. Dazu sind neben geringer Toxizität der Kunststoffe sowie ihrer Abbauprodukte auch Eigenschaften wie einfache Verarbeitbarkeit und hohe mechanische Stabilität erforderlich. Weiters sollten diese Materialien eine hohe Affinität für Zellen, z.B. Osteoblasten, aufweisen, damit sich diese an die Oberfläche des Formkörpers anlagern und die Bildung von körpereigenem Knochenmaterial um den Kunststoff initiieren können. Besonders wünschenswert ist dabei Kunststoffmaterial, das sich im Lauf der Zeit auflöst, dessen Abbauprodukte vom Körper resorbiert werden, und das gleichzeitig durch natürliches Gewebe, z.B. Knochen, ersetzt wird.

Gewisse Erfolge wurden in der Vergangenheit zunächst mit Polymeren auf der Basis von Polymilch- und -glykolsäure sowie Polylactonen und -lactamen erzielt, die jedoch allgemein unvernetzt und somit mechanisch relativ instabil sind und sich für manche Anwendungen zudem zu rasch auflösen (Bulk-Erosion). Um diese Nachteile zu beheben, wurden beispielsweise Copolymere, z.B. Block-Copolymere, mit vernetzbaren Gruppen, z.B. Fumarsäure, hergestellt (siehe z.B. T. Matsuda, M. Mizutani, S. Arnold, Macromolecules 33, 795-800 (2000), und M. Mizutani, T. Matsuda, Journal of Biomedical Materials Research 61(1), 53-60 (2002)). Allerdings stellte sich heraus, dass diese Materialien zahlreiche Nachteile aufwiesen. Zum einen waren die polymerisierbaren Basiszusammensetzungen häufig nur als Schmelze oder Lösung verarbeitbar, was sie nicht nur schwierig in der Handhabung macht, sondern auch hohe Energie- und Materialkosten verursacht, da beträchtliche Wärmemengen vonnöten sind bzw. Lösungsmittel entfernt werden muss. Zum anderen waren die Vernetzungsgeschwindigkeiten gering und die Form- und mechanische Stabilität sowie die Elastizität der Polymere aufgrund geringer Vernetzungsdichten unzureichend, so dass ein Einsatz derselben als künstliches Knochenmaterial praktisch unmöglich war. Eine Verbesserung der Polymerisationsgeschwindigkeit der Capronsäure-Derivate wurde durch die Einführung von Acrylat-Endgruppen erzielt (M. Mizutani, T. Matsuda, Journal of Biomedical Materials Research 62, 395 (2002)), die übrigen Nachteile wurden dadurch jedoch nicht beseitigt.

In der US-Anmeldung 2004/0110439 werden bioverträgliche, vernetzte Proteinfasern für medizinische Anwendungen beschrieben, die aus polymerisierten Derivaten von Biopolymeren, wie z.B. Elastin, Kollagen und Gelatine, gesponnen werden und gegebenenfalls auch eingearbeitete lebende Zellen enthalten können. Auch diese Materialien sind aufgrund mangelnder Steifigkeit und Elastizität jedoch z.B. als Knochenersatz ungeeignet.

Einen ausführlichen Überblick über die Problematik findet sich beispielsweise auch in der WO 2006/108202 der Anmelderin, in der als Lösung derselben Zusammensetzungen auf Acrylatbasis beschrieben werden, die gegebenenfalls Hydrolysate von Biopolymeren wie Gelatine, Keratin, Fibrin oder Casein enthalten können und für generative Fertigungsverfahren, wie z.B. Rapid-Prototyping- oder Rapid-Manufacturing-Verfahren, geeignet sind. Dadurch konnten auf relativ einfache und wirtschaftliche Weise Formkörper erhalten werden, deren mechanische Eigenschaften, einschließlich der Porosität, jenen von natürlichem Knochen ähneln und die - gegebenenfalls nach Oberflächenmodifizierung - gute Zellanhaftung ermöglichen.

Im Zuge weiterführender Forschungen haben die Erfinder jedoch herausgefunden, dass die in der WO 2006/108202 beschriebenen Kunststoffe den Nachteil aufweisen, dass die Abbauprodukte der dortigen Formkörper auf Polyacrylatbasis, d.h. Acrylate, ungünstig hohe Toxizität, auch aufgrund von Restmonomeren, für Zellen aufweisen, so dass bereits angelagerte Zellen absterben können bzw. die Anlagerung weiterer Zellen behindert wird, sobald der biologische Abbau des Kunststoffs im Körper einsetzt.

In JP-A-09143194 wird von Tokiwa Yutaka et al. die Herstellung von Vinyloxycarbonylalkanoyl-Derivaten von Zuckern (d.h. von gemischten Vinyl-Zucker-Estern von Alkandisäuren) durch enzymatische Glykosylierung, ausgehend von Divinylestern aliphatischer Dicarbonsäuren und dem entsprechenden Zucker in Gegenwart von Proteasen von Streptomyceten oder Bacilli beschrieben. Als einzige Beispiele für den Zucker und den Divinylester werden Maltose bzw. Divinyladipat offenbart. Das Hauptaugenmerk liegt dabei auf dem Herstellungsverfahren. Eine mögliche Eignung der so hergestellten Verbindungen als Monomere zur Herstellung biologisch abbaubarer Polymere zum Einsatz in der Polymerchemie und in der Medizin wird zwar allgemein erwähnt, es finden sich jedoch weder Angaben über solche Verbindungen enthaltende polymerisierbare Zusammensetzungen noch über die Eigenschaften von daraus hergestellten Polymeren oder gar von dreidimensionalen Körpern aus diesen Polymeren.

Derselbe Erfinder beschreibt im späteren JP-Patent Nr. 2000-086806 Details zur biologischen Abbaubarkeit von Homopolymeren derartiger Verbindungen, explizit von Vinyladipoylglucose-Homopolymer, durch Mikroorganismen,

die in einem Fall bei über 70 % liegen soll. Die dabei entstehenden Spaltprodukte werden jedoch nicht erwähnt.

Wiederum derselbe Erfinder offenbart im JP-Patent Nr. 2003-321624 Beschichtungen aus ähnlichen Materialien, die gute Haftung auf verschiedenen Oberflächen, wie z.B. Kunststoffen, Metallen, Papier, Gummi, Fasern usw., biologische Abbaubarkeit und Affinität für Proteine, Nucleoside, Nucleotide, Nucleinsäuren usw. aufweisen sollen, wodurch die Beschichtungen etwa zu deren Detektion eingesetzt werden können. Erneut wird nur Divinyladipat als Beispiel für den Divinylester als Ausgangsmaterial erwähnt. Die Beschichtungen werden durch Eintauchen von Gegenständen, wie etwa eines Films aus Polymilchsäure, in eine wässrige Lösung des Vinyladipoylzuckers in Gegenwart eines Eisen(II)-sulfat/Wasserstoffperoxid-Katalysatorsystems erzeugt. Einsatzmöglichkeiten solcher Beschichtungen außer zur Detektion verschiedener Zellkomponenten werden nicht erwähnt.

In JP-A-2001-316465 wird die Herstellung von wasserlöslichen linearen Polyestern aus Zuckeralkoholen und aliphatischen Dicarbonsäuren oder Derivaten davon durch enzymatische Katalyse mit Lipase beschrieben. Als mögliche Ausgangsprodukte werden Divinyladipat und Divinylsebacat erwähnt, aus denen - offenbar durch enzymatische Umesterung unter Abspaltung von Vinylalkohol - lineare Polyester des jeweiligen Zuckers mit Adipin- oder Sebacinsäure erzeugt werden.

Eine ähnliche Vorgangsweise wird auch in JP-A-11276188 offenbart, wo aus Divinylsebacat in Gegenwart von Lipase produzierenden Alcaligenes-Bakterien "polymere Zuckerester", d.h. offenbar erneut Polyester aus Zucker und Dicarbonsäure, hergestellt werden.

Schon seit längerer Zeit wird die Eignung von biodegradablen Polymeren auch für die Rekonstruktion von weichen Binde- und Stützgeweben (Blutgefäße, Herzklappen, Bauchdecke usw.) untersucht. Wie beim Knochen wird eine vollständige Auflösung des prothetischen Materials ohne Funktionsverlust des Implantates bei gleichzeitiger Neubildung von organspezifischem Gewebe angestrebt. Als Ausgangsmaterialien wurden bisher natürliche (z.B. Kollagen, Elastin) und künstliche Polymere (z.B. Polyglykolsäure, Polymilchsäure, Polydioxanon) zur Implantatherstellung eingesetzt. Als Probleme erweisen sich aber nach wie vor die schwer kontrollierbare Graftdegradation und das damit einhergehende Auftreten von Aneurysmen. Weiters kommt es bei vielen Implantaten durch das Biomaterial zur Induktion einer Fremdkörperreaktion, die eigentlich durch den raschen Abbau/Umbau des Materials im Körper verhindert werden sollte. Siehe L. Xue, H.P. Greisler, "Biomaterials in the development and future of vascular grafts", J. Vasc. Surg. 37, 472-480 (2003).

Zu Polymerisationszwecken sind in der Literatur auch Vinylcarbonate und -carbamate bekannt. So beschreibt die WO 93/18070 A1 (die der EP 629.214 B1 entspricht), biologisch abbaubare, unvernetzte Polymere mit geringer oder keinerlei Wasserlöslichkeit, die in Seitenketten lipophile Methylendiester-Gruppen der Formel:

$$\text{Polymer-(L)-(O)}_m\text{-CO-O-C(R}^1\text{R}^2\text{)-O-CO-(O)}_n\text{-R}^3$$

enthalten, worin m und n = 0 oder 1 sind, welche ihrerseits mittels Esterase-Enzym abspaltbar sind, um ein wasserlösliches Restpolymer zu ergeben. L steht für einen optionalen, lipophilen Linker. Bei Ausführungsformen mit m = 1 sind Carbonate in der Seitenkette enthalten. Bei einer von zahlreichen Ausführungsformen kann das Polymer auch Polyvinylalkohol sein, so dass sich - wenn der Linker L fehlt - Polyvinylakohol umfassende Polymere konstruieren lassen. Allerdings sind sämtliche Ausführungsformen der WO 93/18070 A1 völlig unvernetzt, sodass dort ausschließlich lineare Polymere beschrieben werden, die nur geringe bis mäßige mechanische Festigkeit aufweisen können, was beispielsweise eine Verwendung als Knochenersatz ausschließt.

Die WO 2003/37944 beschreibt verschiedene mit Vinylcarbonat endverkappte Polysiloxane mit fluorierten Seitenketten die zusammen mit 3-[Tris(trimethylsiloxy)silyl]-propylvinylcarbonat und mit N-Vinylpyrrolidon zu Hydrogelen copolymerisiert werden, welche als Kontaktlinsenmaterial dienen. Die so erhaltenen Hydrogele sind zwar vernetzt, biologische Abbaubarkeit wird jedoch für solche Copolymere nicht nur nicht erwähnt, sondern ist sogar gänzlich unerwünscht, um für Kontaktlinsen geeignet zu sein.

Auch in der WO 2006/71388 werden Polysiloxane beschrieben, die als Präpolymere zur Herstellung biomedizinischer Vorrichtungen (vor allem von Kontaktlinsen) dienen sollen und Carbonat- bzw. Carbamat-Gruppen in der Kette enthalten können. Konkret entsprechen diese Präpolymere der Formel:

$$\text{M(*Dii*PS)}_x\text{*Dii*M}$$

worin M ein polymerisierbarer ethylenisch ungesättigter Rest ist, Dii ein zweiwertiger Rest einer Diisocyanatverbindung ist, PS ein zweiwertiger Rest eines Polysiloxandiols oder -diamins ist, x zumindest 2 ist und * für eine zweiwertige Gruppe der Formel- NH-CO-NH-, -NH-COO- oder -OCO-NH- steht. Die Gruppierungen M und PS können in der Kette Carbonat-, Ureido- oder Urethan- oder auch Ether-Gruppen enthalten. In jeweils einer der mannigfaltigen möglichen Ausführungsformen kann M eine endständige Vinylcarbonat- oder Vinylcarbamat-Gruppe aufweisen. Als Vorteil der Gegenwart von hydrophilen Carbonat-, Carbamat- oder Ureido-Gruppen wird nicht Spaltbarkeit, sondern eine Erhöhung der Verträglichkeit mit hydrophilen Comonomeren erwähnt. Als mögliches Co-Monomer wird 2-Methacryloyloxyethyl-vinyl-carbonat

erwähnt. Der Vorteil der aus diesen Präpolymeren erhaltenen Kunststoffe ist neben hohem Zugelastizitätsmodul deren hohe Sauerstoffpermeabilität, wie dies für eine Verwendung als Kontaktlinsen erforderlich ist. Biologische Abbaubarkeit ist jedenfalls erneut unerwünscht. Auch die WO 2006/71479 und WO 2001/74932 derselben Anmelder beschreiben die Herstellung bzw. Beschichtung von Kontaktlinsen, wobei erneut 2-Methacryloyloxyethyl-vinyl-carbonat sowie N-(Carboxyethyl)vinylcarbamat als mögliche Comonomere offenbart werden.

Keines der aus dem Stand der Technik bekannten Polymere oder Copolymere wäre demnach für eine Verwendung für hochstabile Materialien, wie z.B. als Knochenersatz- oder Zahlfüllmaterial, geeignet.

Ziel der Erfindung war daher die Bereitstellung einer verbesserten Zusammensetzung zur Herstellung von bioverträglichen Kunststoffen, die als Körperimplantate, insbesondere als Knochenersatz oder als Zahnfüllmaterial, eingesetzt werden können.

OFFENBARUNG DER ERFINDUNG

Die Erfindung löst die obige Aufgabe in einem ersten Aspekt durch Bereitstellung der Verwendung einer durch Polymerisation härtbaren Zusammensetzung zur Herstellung biologisch abbaubarer, bioverträglicher, vernetzter Polymere, vorzugsweise solcher auf Basis von Polyvinylalkohol, für Körperimplantate sowie als Gewebe-Stütz- und -Regenerations-Material, wobei die Zusammensetzung Folgendes umfasst:

a) 5 bis 100 Gew.-% eines oder mehrerer Vinylester-Monomere, die unabhängig voneinander aus Verbindungen der allgemeinen Formeln (I) bis (III) ausgewählt sind:

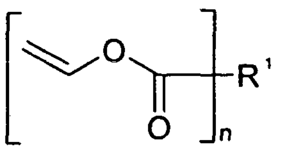
(I)

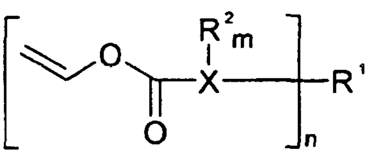
(II)

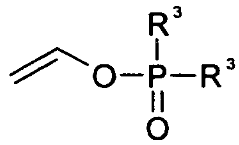
(III)

worin
X ein aus Sauerstoff, Schwefel, Stickstoff und Phosphor ausgewähltes Heteroatom ist;
die n jeweils unabhängig voneinander 1 bis 1000, vorzugsweise 1 bis 50, noch bevorzugter 1 bis 20, noch bevorzugter 1 bis 10, insbesondere 1 bis 3, sind, wobei zumindest 20 % der $n \geq 2$ sind;
die $R^1$ jeweils unabhängig voneinander ausgewählt sind aus

i) Wasserstoff, unverzweigten, verzweigten oder zyklischen, gesättigten oder ungesättigten, n-wertigen Kohlenwasserstoffresten mit 1 bis 30, vorzugsweise 3 bis 25, noch bevorzugter 4 bis 20, insbesondere 5 bis 15, Kohlenstoffatomen, die gegebenenfalls ein oder mehrere, aus Sauerstoff, Schwefel, Stickstoff und Phosphor ausgewählte Heteroatome innerhalb der Kette und/oder am Kettenende aufweisen und gegebenenfalls mit einem oder mehreren Substituenten, ausgewählt aus -OH,

- COOH, -CN, -CHO und =O substituiert sind, und

ii) n-wertigen Resten biologisch abbaubarer, bioverträglicher Oligo- und Polymere, ausgewählt aus Polysacchariden, Polypeptiden, Polyamiden, Polyestern, Polycarbonaten, Polyethern und Fettsäurederivaten;

m eine ganze Zahl von 0 bis 4 ist;
die Reste $R^2$ aus Wasserstoff, -OH, =O und den Optionen für $R^1$ ausgewählt sind; und
die Reste $R^3$ aus Wasserstoff, -OH und den Optionen für $R^1$ ausgewählt sind;

b) 0 bis 50 Gew.-% eines oder mehrerer ethylenisch ungesättigter Comonomere, ausgewählt aus (Meth)acrylsäure-, Maleinsäure-, Fumarsäure-, Vinylpyrrolidon- und $\alpha$ -Olefin-Monomeren;

c) 0 bis 10 Gew.-% eines oder mehrerer Polymerisationsinitiatoren, ausgewählt aus thermischen Initiatoren und Photoinitiatoren; und

d) 0 bis 95 Gew.-% eines oder mehrerer Lösungsmittel, ausgewählt aus Wasser, niederen Alkoholen, Ether-, Keton-, Ester-, Amid- und Kohlenwasserstoff-Lösungsmitteln.

Die Zusammensetzungen umfassen demnach als Hauptkomponente, abgesehen von allfälligem Lösungsmittel:

- einen oder mehrere Carbonsäurevinylester der allgemeinen Formel (I) und/oder
- einen oder mehrere Kohlensäure-, Thiokohlensäure- oder Carbaminsäurevinylester bzw. eine oder mehrere Vinyloxycarbonylphosphorverbindungen, vorzugsweise Vinyloxycarbonyl-Derivate von Säuren des Phosphors, insbesondere von Phosphonaten, der allgemeinen Formel (11) und/oder
- einen oder mehrere Vinylester einer Säure des Phosphors, vorzugsweise ein oder mehrere Vinylphosphate, der allgemeinen Formel (III)

als polymerisierbare Monomere, die nachstehend allesamt als "Vinylester" bezeichnet werden.

Da die Zusammensetzung somit auf Vinylester-Derivaten basiert, bildet sich bei der Polymerisation derselben eine zum Teil, vorzugsweise aber vorwiegend aus Polyvinylalkohol bestehende Polymerkette. Im Zuge des biologischen Abbaus des Polymers im Körper werden daher primär Polyvinylalkohol sowie - zumindest intermediär - die entsprechenden freien Säuren bzw. Partialester, die nachstehend der Einfachheit halber beide als "Säuren" bezeichnet werden, und/oder Reste von biologisch abbaubaren Oligo- und Polymeren gebildet. Polyvinylalkohol ist ein nichttoxisches Polymer, das häufig in Arzneimittelformulierungen anzutreffen ist, und wird ohne Schäden für den Körper ausgeschieden.

Als Säuren werden im Falle von Monomeren der Formel (I) beispielsweise Fettsäuren, Zuckersäuren oder Aminosäuren gebildet, wobei erfindungsgemäß insbesondere solche zum Einsatz kommen, die als Bestandteile der Nahrung ebenfalls weitgehend unbedenklich sind. Ähnliches gilt für die - in der Folge zusammenfassend als "Biopolymere" bezeichneten - Oligo- und Polymere als Komponente a)ii): Auch hierfür werden biologische Substanzen oder leicht abbaubare Kunststoffe eingesetzt, die gut verträglich und für den Organismus unschädlich sind. Darauf wird nachstehend noch näher eingegangen.

Im Falle von Monomeren der Formel (II) bilden sich beispielsweise (Thio-)Kohlensäurehalbester oder Carbaminsäuren, die jedenfalls an Heteroatome gebundene Carboxylgruppen aufweisen, die als solche instabil sind und spontan decarboxylieren. Dies bedeutet, dass an den Resten $R^1$ keine freien Carboxylgruppen zurückbleiben, sondern als saure Verbindung lediglich $CO_2$ anfällt, das über die Lungen ausgeschieden wird. Da keine sauren Komponenten lokal verbleiben, sind Verbindungen der Formel (II) bevorzugte Monomere in den Zusammensetzungen.

Bei Monomeren der Formel (III) fallen bei der Zersetzung der Polymere im Körper ausschließlich Säuren des Phosphors, vorzugsweise Phosphate, an, die ebenfalls weitestgehend unbedenklich und teilweise sogar zum Aufbau körpereigener Substanzen erforderlich sind.

Aus einer obigen Zusammensetzung kann somit ein Kunststoff erhalten werden, der aufgrund der Gegenwart von zumindest 20 Mol-% polyfunktioneller Vinylester-Monomere (da 20 % der $n \geq 2$ sind) stabil vernetzt ist und der aufgrund seiner, wenn überhaupt, äußerst geringen Toxizität problemlos als Implantat im Körper eingesetzt werden kann. Durch geeignete Wahl der Parameter wie etwa des Verhältnisses zwischen mono- und polyfunktionellen Vinylestermonomeren und des Monomergehalts, können sowohl Hydrogele (z.B. aus Zusammensetzungen mit geringem Monomergehalt in Wasser) bzw. so genannte "PEG-o-Gele" (d.h. mit Polyethylenglykol als Lösungsmittel) als auch steife, elastische Körper (z.B. aus lösungsmittelfreien Zusammensetzungen mit hohem Anteil an polyfunktionellen Monomeren) gebildet werden, die somit beispielsweise als Gewebe-, Knorpel- oder Knochenersatz oder auch als Zahnfüllmaterial Anwendung finden können. Weiters können die so erhaltenen Polymere als Gewebestützen, z.B. für Herzklappen, als Grundmaterial für Shunts und Stents sowie als Kleber und Verschlüsse (z.B. Patches) für verletzungs- oder genetisch bedingte Gewebeschäden eingesetzt werden. Weiters sind solche Formulierungen auch geeignet für die Herstellung von Beschichtungen verschiedener Substrate, z.B. für Medizinprodukte, aber auch überall dort, wo geringe Toxizität der Monomere und der Polymere erwünscht ist, z.B. im Lebensmittelkontakt.

Die Anzahl an Vinylestergruppierungen in der Zusammensetzung wird durch entsprechende Wahl des Parameters n gesteuert. Werden Vinylester von Biopolymeren mit einem hohen Molekulargewicht, z.B. über 10.000 oder sogar über 1.000.000 g/mol, eingesetzt, können je nach Substitutionsgrad durchaus bis zu 1.000 reaktive Stellen, d.h. Vinylestergruppen, am Polymerrückgrat vorliegen, beispielsweise im Falle von Stärke als Biopolymer. Aufgrund der für manche Anwendungen möglicherweise zu hohen Vernetzungsdichte sowie zur Erhöhung der Auflösungsgeschwindigkeit der Polymere im Körper werden jedoch auch im Falle von Biopolymeren als Reste $R^1$ weniger reaktive Stellen, d.h. bis zu 50, bis zu 20 oder nur bis zu 10 Vinylestergruppen, pro Monomermolekül bevorzugt. Speziell, wenn keine Biopolymere, sondern Monomere bzw. kurzkettige Oligomere (z.B. Dimere) als $R^1$ zum Einsatz kommen, sind vorzugsweise nur bis zu 10, noch bevorzugter nur bis zu 3, Vinylestergruppen im Monomer-Molekül vorhanden.

Der Wert des Parameters m in Formel (I), d.h. die Anzahl an Substituenten auf dem Heteroatom X mit Ausnahme des Vinylester- und des $R^1$-Rests, kann zwischen 0 und der um zwei reduzierten Wertigkeit des Heteroatoms X variieren, d.h. für Sauerstoff ist m = 0, für Stickstoff ist m = 1, für Schwefel ist m = 0 bis 4, und für Phosphor ist m = 0 bis 3. Ist ein

mehrwertiger Rest, wie z.B. =O, an das Heteroatom gebunden, verringert sich die Anzahl der weiteren möglichen Substituenten $R^2$ natürlich entsprechend der Wertigkeit desselben.

Weiters können zwei oder mehrere der Reste $R^1$ und $R^2$ miteinander verbunden sein, um so Ringstrukturen zu bilden, in denen X ein Ringatom darstellt. Wie die Formel (I) ausdrückt, können sowohl mehrere Vinylester/Heteroatom-Gruppierungen an einen Rest $R^1$ gebunden sein als auch ein oder mehrere Heteroatome X mehr als einen aus den Optionen für $R^1$ ausgewählten Substituenten $R^2$ aufweisen.

Die Anzahl der Kohlenstoffatome des Rests $R^1$ als Komponente a)i), der kein Biopolymer ist, hängt unter anderem vom jeweiligen Wert für n ab. Obgleich sowohl sehr kurzkettige Verbindungen wie Divinyl(thio)carbonat oder Vinylcarbamat als auch langkettige oder stark verzweigte bzw. durch zyklische Strukturen unterbrochene Reste mit bis zu 30 C-Atomen eingesetzt werden können, sind derartig kurze bzw. lange/verzweigte Reste erfindungsgemäß nicht bevorzugt. Sehr niedermolekulare Verbindungen sind aufgrund ihrer relativen Flüchtigkeit schwieriger zu handhaben und langkettige oder stark verzweigte Reste im Körper mitunter schwerer abbaubar. Daher sind Reste $R^1$ mit 3 bis 25 Kohlenstoffatomen eher bevorzugt, und solche mit 4 bis 20, insbesondere 5 bis 15, Kohlenstoffatomen prinzipiell noch bevorzugter, wenn dies auch, wie erwähnt, vom Wert für n abhängt. Ist $R^2$ ein aus den Optionen für $R^1$ ausgewählter Rest, so handelt es sich dabei im Hinblick auf die mechanischen und die Polymerisationseigenschaften vorzugsweise um kurzkettige Reste, z.B. Niederalkyl- oder -alkoxy-Reste.

Vinylester-Monomere der Formel (I) sind somit vorzugsweise aus Vinylestern von aliphatischen Carbonsäuren und Hydroxycarbonsäure mit 4 bis 20 Kohlenstoffatomen, Zuckersäuren, Aminosäuren sowie Polymeren und Copolymeren der obigen Säuren ausgewählt, noch bevorzugter aus ein- und mehrfachen Estern der folgenden Säuren und deren Derivaten: Bernsteinsäure, Adipinsäure, Fumarsäure, Citronensäure, Weinsäure, Asparaginsäure, Oxoglutarsäure, Glutaminsäure, Schleimsäure, Ethylendiamintetraessigsäure, Butantetracarbonsäure, Cyclopentantetracarbonsäure, Polyglutaminsäure, Polyasparaginsäure, Hyaluronsäure, Polymilchsäure, Polyglykolsäure und Poly(lactid-co-glykolid).

Wenn $R^1$ den Rest eines Biopolymers darstellt, kann dieses beispielsweise aus den folgenden ausgewählt werden: Polyethylenglykol, Gelatine, Chitosan, Cellulose, Amylose und Glykogen. Diese Auswahl gewährleistet besonders gute Verträglichkeit der Abbauprodukte eines aus der Zusammensetzung hergestellten Polymers sowie leichte Verfügbarkeit oder Zugänglichkeit der Ausgangssubstanzen für die Zusammensetzung.

Bei den Vinylester-Monomeren der Formel (III), d.h. den Vinylestern von Säuren des Phosphors, also von Phosphor-, Phosphon- oder Phosphinsäuren, sind die $R^3$ vorzugsweise OH-, Niederalkyl- oder Alkoxygruppen oder Biopoly- oder -oligomere. In manchen bevorzugten Ausführungsformen sind beide Reste $R^3$ Alkoxygruppen, wovon eine besonders bevorzugt eine weitere Vinyloxygruppe darstellt, sodass das Monomer ein Divinylester der jeweiligen Säure des Phosphors ist, der in der Zusammensetzung als Vernetzer dient. Die Vinylester-Monomere der Formel (III) können in anderen bevorzugten Varianten Vinylester von Nucleosiden, Nucleotiden oder Nucleinsäuren sein, um bei der Zersetzung vom Körper verwertbare Produkte zu liefern.

In den Zusammensetzungen kann jeweils nur ein Vinylester-Monomer einer der Formeln (I) bis (III) enthalten sein, dass dann jedoch ein zumindest bifunktionelles Monomer, d.h. ein Divinylester, sein muss, um bei der Polymerisation den erforderlichen Mindestvernetzungsgrad zu ergeben. Vorzugsweise sind daher mehrere verschiedene Vinylester-Monomere in den Zusammensetzungen enthalten, beispielsweise zumindest ein mono- und zumindest ein di- oder höherfunktionelles Monomer, da so der Vernetzungsgrad einfacher zu steuern ist. Bei Gegenwart mehrerer verschiedener Vinylester-Monomere können diese allesamt nur einer oder auch unterschiedlichen der Formeln (I) bis (III) entsprechen. Das heißt, es können beispielsweise Kombinationen von Vinylcarboxylaten, -carbonaten bzw. -carbamaten und -phosphaten in den Zusammensetzungen enthalten sein. Die Auswahl solcher Kombinationen ist nicht speziell eingeschränkt und kann in Abhängigkeit vom jeweiligen Verwendungszweck des daraus herzustellenden Polymers frei gewählt werden, solange bei der Polymerisation die gewünschten Eigenschaften des gehärteten Produkts erzielt werden.

In bevorzugten Ausführungsformen macht das zumindest eine Vinylester-Monomer zumindest 50, noch bevorzugter zumindest 70, insbesondere zumindest 90, Mol-% aller enthaltenen Monomere aus, um bei der Polymerisation einen Kunststoff zu ergeben, der hohe Anteile an Polyvinylalkohol enthält, was die oben beschriebenen Vorteile der Erfindung noch besser zur Geltung bringt.

Vorzugsweise sind weiters zumindest 35, noch bevorzugter zumindest 50, Mol-%, der Vinylester-Monomere der Zusammensetzung di- oder höherfunktionelle, als Vernetzer wirkende Monomere mit $n \geq 2$. Dies bietet - sowohl bei niedrigem als auch bei hohem Gesamtmonomergehalt in der Zusammensetzung - den Vorteil einer ausreichenden Vernetzungsdichte, um Formstabilität sowie gewünschte mechanische Eigenschaften, wie z.B. Härte und Stabilität, zu gewährleisten.

Der Umstand, dass in der Kette bzw. am Kettenende optional weitere Heteroatome vorhanden sein können, trägt der Tatsache Rechnung, dass in biologischen Molekülen der angegebenen Kettenlängen, wie etwa in Zucker(säure)-, Aminosäure- bzw. Peptid- oder Fettsäureresten, aus denen die Vinylester-Monomere hergestellt werden können, häufig Heteroatome anzutreffen sind. Dasselbe gilt für die gegebenenfalls vorhandenen Substituenten, Unsättigungen und Verzweigungen. Die optionalen Substituenten können aber auch dazu dienen, die Anlagerung von Zellen an der

Oberfläche eines aus der Zusammensetzung hergestellten Polymers zu fördern, was nachstehend noch näher ausgeführt wird.

Die Vinylester-Monomere der Zusammensetzungen sind entweder im Handel erhältlich, oder sie können gemäß literaturbekannten Verfahren oder entsprechend den hierin in den nachfolgenden Synthesebeispielen offenbarten Verfahren hergestellt werden, wobei dem Fachmann klar ist, dass die Reaktionsparameter mitunter entsprechend abzuändern sind, um weitere, hierin nicht beschriebene Verbindungen zu synthetisieren. Beispielsweise kann zur Herstellung von Vinylestern der Formel (II) mit Schwefel als Heteroatom X die in Synthesebeispiel 8 beschriebene Arbeitsvorschrift befolgt werden, wobei anstelle von Ethylenglykol das entsprechende Thiol oder z.B. eine HS-Gruppen enthaltende Aminosäure wie Cystein, deren sonstige Funktionalitäten gegebenenfalls geschützt sind, mit Chlorameisensäurevinylester umgesetzt wird. Bei Bedarf kann gegebenenfalls die Reaktionstemperatur (z.B. auf Raumtemperatur) erhöht werden, um die geringere Reaktivität von Thiolen zu kompensieren. Jedes andere Verfahren, das die gewünschten Verbindungen liefert, ist aber ebenso geeignet, wofür beispielsweise folgende Literatur angeführt werden kann.

Carbonate:

R.A. Olofson und J. Cuomo, Tetrahedron Lett. 21 (9), 819-22 (1980), beschreiben die Synthese von Isobutylvinylcarbonat aus Trimethylsilylvinylether und Chlorameisensäureisobutylester unter Verwendung von Benzyltrimethylammoniumfluorid als Katalysator.

R.A. Olofson, Dang Vu Anh; D.S. Morrison und P.F. De Cusati, J. Org. Chem. 55(1), 1-3 (1990), beschreiben eine Einstufensynthese aus Chlor- oder Fluorameisensäureestern und Aldehyden unter Kronenether-Katalyse.

K. Rege, S. Hu, J.A. Moore, J.S. Dordick und S.M. Cramer, J. Am. Chem. Soc. 126(39), 12306-12315 (2004), beschreiben die chemoenzymatische und daher regioselektive Synthese ausgehend von Methylenoximvinylcarbonat und Alkoholen.

Carbamate:

R.A. Olofson, B.A. Bauman und D.J. Wancowicz, J. Org. Chem. 43(4), 752-4 (1978), beschreiben die Herstellung von Vinylcarbamaten aus dem entsprechenden Amin, Phosgen und Di(ethanal-2-yl)quecksilber.

A.J. Duggan und F.E. Roberts, Tetrahedron Lett. 20(7), 595-8 (1979), beschreiben eine Synthese ausgehend vom entsprechenden Amin und von S-Phenylvinylthiocarbonat.

Thiocarbonate:

A.J. Duggan und F.E. Roberts, Tetrahedron Lett. 20(7), 595-8 (1979), beschreiben die Synthese von S-Phenylvinylthiocarbonat aus Thiochlorameisensäure-S-phenylester und Vinylalkohol.

R.A. Olofson und J. Cuomo, J. Org. Chem. 45(12), 2538-41 (1980), beschreiben eine ähnliche Synthese aus aus Thiochlorameisensäure-S-phenylester und Trimethylsilylvinylether.

Weitere mögliche Ausgangssubstanzen für die Herstellung der Vinylester-Monomere umfassen beispielsweise diverse Mono- und Polyalkohole, einschließlich Zucker- und Zuckersäure-Derivate, wie z.B. diverse Glykole, Glycerin, Cyclohexandimethanol, Hexandiol, Hexanol, Butanol, Ethanol, Dodecanol, Trimethylolpropan, Stearyltartrat, Glucose, Ribose, Fructose, Glycerinaldehyd, Dihydroxyaceton, Desoxyribose, Cellobiose, Glucopyranose, Erythrose, Threose, sowie deren Thio-Analoga, Amine und Polyamine, Aminosäuren (vorzugsweise essenzielle), Nucleotide und Nucleotidbasen, Peptide, wie z.B. Jeffamine, Piperidin, Ethylendiamin, Hexamethylendiamin, Diethylentriamin, Triethylentetramin, 1,12-Diamino-4,9-diazadodecan, 1,5,10-Triazadecan, Hexylamin und Dodecylamin, Polymere und Biopolymere, wie z.B. Stärke, Cellulose, Chitosan, Alginat, Hydroxyethylcelluose, Hydroxyethylstärke, Hyaluronat, Gelatine, Casein, Polyvinylalkohol, Polyethylencarbonat, Poly-1,2-propylencarbonat, Polycaprolactondiol, aber auch Zwei- und Drei-Block-Copolymere wie PEG-Caprolacton, PEG-Glykole, PEG-Lactide, PEG-Ethylencarbonate und PEG-Propylencarbonate, sowie verschiedene Verbindungen mit biologischer Aktivität, wie z.B. Salicylsäureethylester, Ascorbinsäure, Ubichinon, Gallussäure, Citronensäure, Curcumin, Retinol, Calciferol, Thiamin, Diaminopyrimidin, um nur einige zu nennen.

Die optionalen Comonomere als Komponenten b) können bei Bedarf zu verschiedensten Zwecken miteinbezogen werden, etwa wiederum zur Oberflächenmodifizierung zur Förderung der Zellanlagerung, zur festen Anbindung bestimmter Komponenten der Zusammensetzung, wie z.B. von Initiatoren oder optionalen Additiven, beispielsweise zur Fixierung an bestimmten Stellen im Molekül, aber auch zur Modifizierung der mechanischen Eigenschaften des polymerisierten Produkts. Bevorzugt werden natürlich auch hierfür bioverträgliche, nichttoxische Verbindungen, in geringen Anteilen können jedoch auch andere, wie z.B. Acryl- und Methacrylsäurederivate, eingesetzt werden. Dies hängt auch von den übrigen Komponenten der Zusammensetzung ab sowie davon, wie und an welchen Positionen diese Comonomere in die Polymerkette eingebaut werden sollen. Vorzugsweise werden die Comonomere als Komponente b) aus (Meth)acrylsäureanhydrid, (Meth)acrylsäureglycidylester, (Meth)acryloyloxybernsteinsäureanhydrid, (Meth)acryloyloxymethylbernsteinsäure-anhydrid, (Meth)acrylsäure-2-oxo-1,3-dioxolanylmethylester, Vinylbernsteinsäureanhydrid, Vinylencarbonat und Maleinsäureanhydrid ausgewählt, da diese Derivate verhältnismäßig gut verträglich sind und/oder leicht Verbindungen mit gewünschten Partnern, wie z.B. Funktionalitäten auf Zelloberflächen, Additiven oder Initiatoren, eingehen können. Ebenfalls geeignet sind unter Ringöffnung radikalisch polymerisierbare Comonomere, wie z.B. zyklische Carbonate, die das Polyvinylalkohol-Rückgrat unterbrechen und im Körper gespalten werden können, um so kürzere Polyvinylalkohol-Ketten bereitzustellen, deren Clearance leichter und rascher vonstatten geht.

In bevorzugten Ausführungsformen der Erfindung weist zumindest eines der Monomere oder Comonomere Funktionalitäten auf, die in der Lage sind, über Haupt- oder Nebenvalenzen, wie z.B. Van-der-Waals-Kräfte oder Wasserstoffbrücken, eine Bindung mit Zelloberflächen bzw. Rezeptoren darauf einzugehen. Dies sorgt einerseits für gute Zellanhaftung am gehärteten Polymer, andererseits können aber auch in bekannter Weise lebende Zellen als "Additive" in die Zusammensetzung einbezogen und über diese Funktionalitäten immobilisiert werden.

Polymerisationsinitiatoren können in der Zusammensetzung enthalten sein, z.B. wenn es sich um eine UV/VIS-härtbare Zusammensetzung handelt. Die Polymerisation kann jedoch auch thermisch oder durch Elektronen- oder Gammastrahlung ohne Initiator ausgelöst werden, was jedoch nicht bevorzugt ist. In bevorzugten Ausführungsformen der Erfindung sind vielmehr 0,1 bis 10, vorzugsweise 0,2 bis 5, noch bevorzugter 0,5 bis 3, Gew.-% zumindest eines Polymerisationsinitiators als Komponente c) enthalten, da die Härtung dadurch kostengünstiger und vollständiger durchgeführt werden kann. Noch bevorzugter ist der zumindest eine Initiator ein Photoinitiator, insbesondere ein UV/VIS-Initiator, was die Zusammensetzung besonders geeignet für Rapid-Prototyping- oder Rapid-Manufacturing-Verfahren macht.

Wie bereits oben erwähnt, kann die Zusammensetzung für manche Anwendungen des fertigen Polymers ein Lösungsmittel enthalten, etwa wenn das gewünschte Produkt ein Hydrogel ist. In vielen Fällen ist jedoch eine lösungsmittelfreie Zusammensetzung bevorzugt, beispielsweise zur Verwendung der Zusammensetzung in Rapid-Prototyping- oder Rapid-Manufacturing-Verfahren. Wenn ein Lösungsmittel zum Einsatz kommt, so ist dieses vorzugsweise Wasser oder ein anderes gut verträgliches, z.B. ein Alkohol, (Poly-)Glykol oder (Pflanzen-)Öl.

Durch optionale Additive können der Zusammensetzung gewünschte Eigenschaften verliehen werden. Deren Menge sind nicht speziell eingeschränkt, solange die Wirkung der Erfindung nicht beeinträchtigt wird. Vorzugsweise sind die Additive aus Polymerisations-Sensibilisatoren und -Inhibitoren, Stabilisatoren, Modifikatoren, Weichmachern, Färbemitteln, bioaktiven Wirkstoffen, Zellen, wie z.B. Osteoblasten und glatten Muskelzellen, Verdickern und Füllstoffen ausgewählt, wodurch einerseits fachübliche Kunststoffzusätze eingearbeitet werden können, andererseits aber auch auf das Verhalten des späteren, gehärteten Produkts Einfluss genommen werden kann. So können etwa in besonders bevorzugten Ausführungsformen die bioaktiven Wirkstoffe aus Arzneimitteln, Proteinen und Liganden von Zelloberflächenrezeptoren ausgewählt werden. Beispielsweise können Thrombozytenaggregationshemmer/Blutgerinnungsinhibitoren oder Immunsuppressiva, aber auch Peptide zur Beeinflussung der Zellvermehrung bzw. Zelldifferenzierung in die Zusammensetzung miteinbezogen und/oder an die Oberfläche des gehärteten Polymers gebunden werden. Weiters fallen zellselektive Proteine wie etwa Antikörper, z.B. Anti-CD34 oder Anti-CD133, die sich über Antigen/Antikörper-Reaktionen an Stamm- bzw. Vorläuferzellen binden können, oder Komplementinhibitoren zur Verhinderung von Entzündungen an der Oberfläche der Polymere, in diese Gruppe. Auch bekannte Zellanhaftungsverbesserer können eingearbeitet und/oder oberflächlich gebunden sein, wie beispielsweise Carboxymethyldextrane, Proteoglykane, Kollagen, Gelatine, Glucosaminoglykane, Fibronectin, Lectine, Polykationen sowie natürliche und synthetische biologische Zellhaftmittel, wie z.B. RGD-Peptide. So kann einerseits erneut für gute Zellanhaftung gesorgt werden, andererseits kann durch den kombinierten Einsatz von Arzneimitteln das aus der Zusammensetzung erhaltene Polymer auch als Arzneimittelträger dienen - zusätzlich oder auch anstelle seiner Funktion als Ersatz oder Stützmaterial für bestimmte Körpergewebe.

Weitere mögliche Füllstoffe umfassen Tricalciumphosphat, $Ca_3(PO_4)_2$, und Hydroxyapatit, die einerseits als Calciumquelle für den Knochenaufbau dienen, andererseits aber auch die Zellanhaftung verbessern, sowie verschiedenste organische Füller, worunter beispielsweise auch autologes Serum oder Plasma des Transplantatempfängers fällt.

Ein oder mehrere Additive können auch kovalent an Monomere oder Comonomere gebunden sein, z.B. an eines oder mehrere der obigen leicht derivatisierbaren Comonomere, wie zuvor besprochen, etwa als Ester oder über andere Funktionalitäten der (Co-)Monomere. Dies kann nicht nur eine einheitlichere Verteilung des Additivs gewährleisten, als sie möglicherweise bei bloßer physikalischer Vermengung mit den übrigen Komponenten der Zusammensetzung erzielbar wäre, sondern beispielsweise auch eine Anbindung einer bestimmten Komponente ausschließlich an die Oberfläche des Polymers, wenn das entsprechende Additiv erst zugesetzt wird, nachdem bereits eine Vorhärtung der übrigen Komponenten stattgefunden hat. Besonders bevorzugt ist daher zumindest ein solches, kovalent an Monomere oder Comonomere gebundenes Additiv ein bioaktiver Wirkstoff, wie z.B. ein Arzneimittel oder ein Mittel zur Förderung der Zellanhaftung, da ein solcher Wirkstoff seine Funktion überwiegend an der Oberfläche des fertigen Kunststoffs zu erfüllen hat.

Durch die Erfindung wird ein biologisch abbaubares, bioverträgliches, vernetztes Polymer, vorzugsweise auf Basis von Polyvinylalkohol, bereitgestellt, das aus einer oben beschriebenen Zusammensetzung in gehärteten Zustand

besteht. Vorzugsweise weist ein solches Polymer an seiner Oberfläche Funktionalitäten auf, die in der Lage sind, über Haupt- oder Nebenvalenzen, wie z.B. Van-der-Waals-Kräfte oder Wasserstoffbrücken, eine Bindung mit Zelloberflächen bzw. Rezeptoren darauf einzugehen, um die Zellanhaftung zu fördern. So kann beispielsweise zumindest einer der zuvor genannten, bekannten Zellanhaftungsverbesserer vorzugsweise an die Oberfläche des Polymers gebunden sein. Die Form des Polymers ist nicht speziell eingeschränkt. So kann es beispielsweise als Strukturkörper, als Beschichtung auf einem Substrat oder als Folie, aber auch als Hydrogel oder "PEG-o-Gel" vorliegen.

Die vorliegende Erfindung betrifft in bevorzugten Ausführungsformen die Herstellung eines biologisch abbaubaren, bioverträglichen, vernetzten Polymers durch thermisches oder photochemisches Polymerisieren einer oben definierten Zusammensetzung. In manchen bevorzugten Ausführungsformen kann ein Teil der Zusammensetzung vorgehärtet werden, wonach erst der Rest der Zusammensetzung zugesetzt und das Gemisch ausgehärtet wird. Dies ermöglicht die gezielte Bindung mancher Bestandteile der Zusammensetzung an der Oberfläche. Gegebenenfalls kann das so erhaltene Polymer, beispielsweise zur Nachhärtung, Entfernung oder Deaktivierung von überschüssigen Additiven oder Rest(co)monomeren oder zur Modifizierung der Oberfläche oder der mechanischen Eigenschaften, aber auch zur Sterilisation für seinen Einsatz als Transplantat, nachbehandelt werden. Nachbehandlungen können auch Wärmebehandlung, Extraktion, Umfällung oder Oberflächenbehandlung, z.B. -imprägnierung, umfassen.

In der erfindungsgemäßen Verwendung kann die Polymerisation, wie oben erwähnt, thermisch oder photochemisch initiiert werden. Photochemisch initiierte Polymerisation wird dabei vorzugsweise in einem generativen Fertigungsverfahren, wie z.B. durch Rapid-Prototyping oder Rapid-Manufacturing, durchgeführt. Dadurch können auch komplizierte Strukturen, wie z.B. jene von Knochen oder Knochenstücken, rasch, relativ kostengünstig sowie äußerst maßgetreu nachgebildet werden. Allerdings eignen sich die Zusammensetzungen aufgrund ihrer geringen Toxizität auch zur Härtung in vivo nach direktem Auftrag derselben auf geschädigtes Gewebe. Sie können jedoch beispielsweise auch in einem gegebenenfalls abbaubaren Beutel oder dergleichen in den Körper eingebracht, in die gewünschte Form gebracht und danach in vivo oder ex vivo gehärtet werden.

Die Erfindung wird anhand der nachstehenden, der Veranschaulichung dienenden und nicht als Einschränkung zu verstehenden Beispiele detaillierter beschrieben.

BEISPIELE

Nachstehend sind die in den Ausführungsbeispielen eingesetzten chemischen Verbindungen zusammen mit den dafür verwendeten Abkürzungen tabellarisch angegeben.

Die hochgestellten Buchstaben a) bis e) geben die kommerziellen Bezugsquellen der Ausgangs- und Vergleichsverbindungen an und stehen für folgende Lieferanten:

a): TCI Europe
b): Ivoclar Vivadent
c): Cognis (Photomer4006 F)
d): Sartomer (Sartomer 415)
e): Sigma Aldrich

| Bezeichnung | Struktur |
|---|---|
| HVE[a)] (Hexansäurevinylester) | 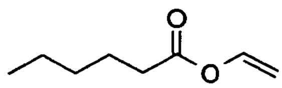 |
| DVE[a)] (Decansäurevinylester) | 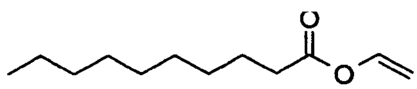 |
| PAVE (N-Acetylphenylalaninvinylester) | 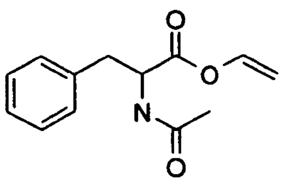 |

(fortgesetzt)

| Bezeichnung | Struktur |
|---|---|
| AVE[a)] (Adipinsäuredivinylester) | 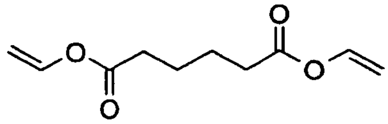 |
| KVE (Korksäuredivinylester) | 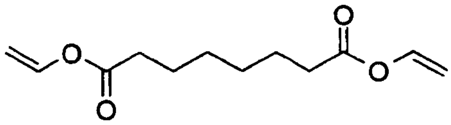 |
| SEVE (Sebacinsäuredivinylester) | 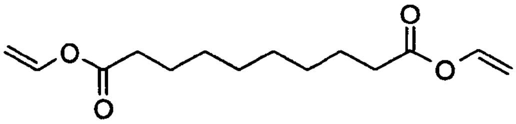 |
| DVMPL (Diethylenglykol-bis[O-(O'-vinylmaleinoyl)polylactat]) | 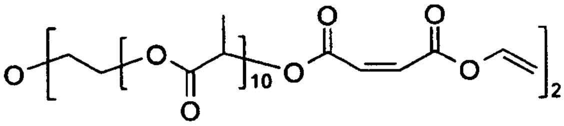 |
| TFVE (Trimerfettsäuretrivinylester) | 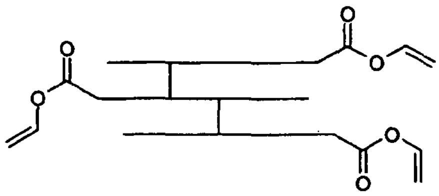 |
| TUVE (w,w'-3,6,9-Trioxaundecandisäuredivinylester) | 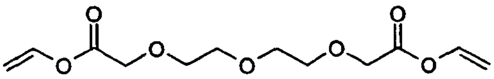 |
| EGDVC (Ethylenglykol-bis(vinylcarbonat)) | 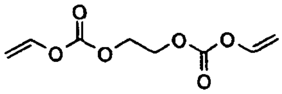 |
| BDDVC (1,4-Butandiol-bis(vinylcarbonat)) | 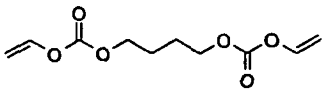 |
| HDDVC (1,6-Hexandiol-bis(vinylcarbonat)) | 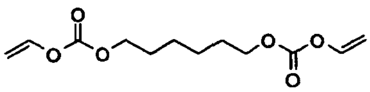 |
| GTVC (Glycerin-tris(vinylcarbonat)) | 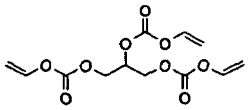 |
| DEGDVC (Diethylenglykol-bis(vinylcarbonat)) | 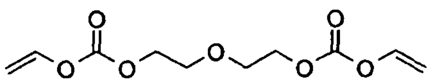 |

(fortgesetzt)

| Bezeichnung | Struktur |
|---|---|
| PEGDVC (Polyethylenglykol(400)-bis(vinylcarbonat)) | 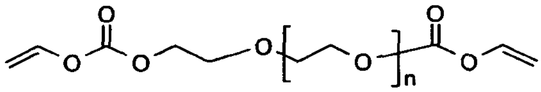 |
| CEVC (2-Cyanoethylvinylcarbonat) | 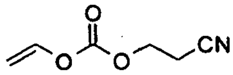 |
| EVC (Ethylvinylcarbonat) | 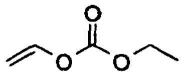 |
| RiTVC (Ricinusöl-tris(vinylcarbonat)) | 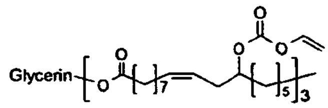 |
| HRiTVC (hydriertes Ricinusöl-tris-(vinylcarbonat)) | 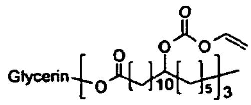 |
| DEG(PLAVC)$_2$ (Diethylenglykol-bis-[O-(O'-vinyloxycarbonyl)polylactat]) | 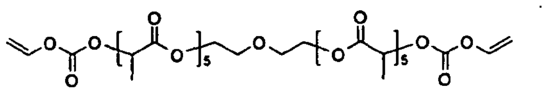 |
| DMEDDVCA (N,N'-Dimethylethylen-diamin-bis(vinylcarbamat)) | 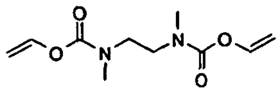 |
| PDVCA (Piperazin-bis(vinylcarbamat)) | 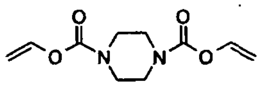 |
| JAVM (3,3'-Ethylendioxy-bis(propylamin)divinylcarbamat) | 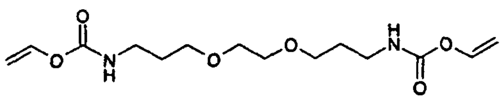 |
| EAVM (Bis[w-aminopolyethylenglykol(500)]amin-tris(vinylcarbamat)) | 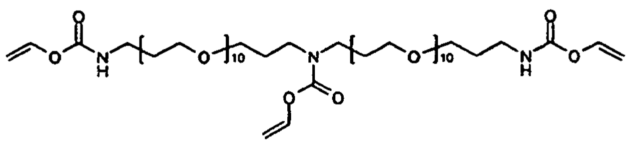 |
| SMEVCA (Sarkosinmethylester-vinylcarbamat) | 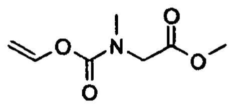 |
| MHADVC (N,O-Bis(vinyloxycarbonyl)-N-methylhyd roxylamin) | 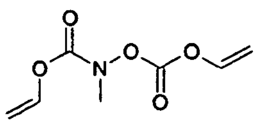 |

(fortgesetzt)

| Bezeichnung | Struktur |
|---|---|
| MVCA (N-Methoxyvinylcarbamat) | 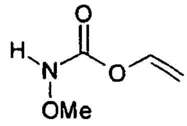 |
| AMVCA (N-Acryloyl-N-methoxyvinylcarbamat | 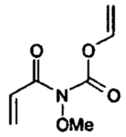 |
| VCPDE (Vinyloxycarbonyl-phosphonsäurediethylester) | 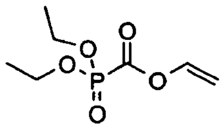 |
| EPEVC (2-Diethoxyphosphoryloxy-ethylamin-vinylcarbamat) | 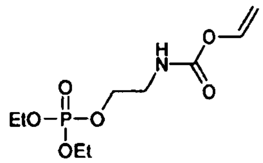 |
| EBVCAEP (Ethyl-bis[2-(vinyloxy-carbonylamino)ethyl]phosphat) | 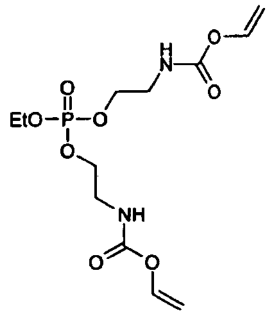 |
| DEVP (Diethylvinylphosphat) | 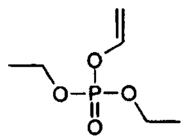 |
| DVEP (Divinylethylphosphat) | 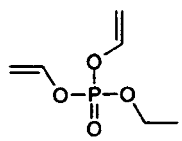 |
| TVP (Trivinylphosphat) | 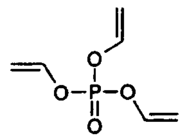 |

| Referenzen: Acrylate | |
|---|---|
| HDDA[c)] (1,6-Hexandioldiacrylat) | 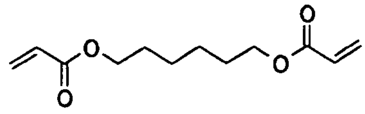 |
| TTA[c)] (Trimethylolpropantriacrylat) | 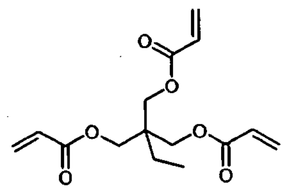 |
| ETA[d)] (ethoxyliertes (20) TTA, MG 1200) | 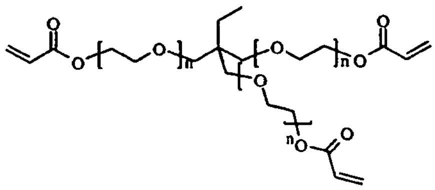 |
| PEGDA[e)] (Polyethylenglykoldiacrylat, MG 800) | 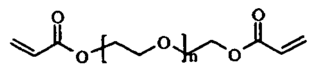 |
| **Referenzen: Methacrylate** | |
| BDMA[e)] (1,4-Butandioldimethacrylat) | 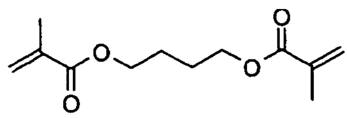 |

<u>Synthesebeispiel 1: Synthese von Sebacinsäuredivinylester (SEVE)</u>

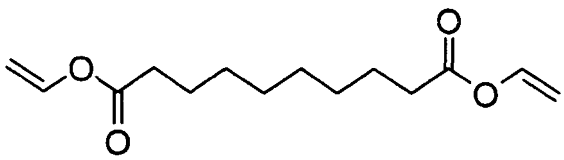

In einem 250-ml-Dreihalskolben wurden 15 g (74,2 mmol) Sebacinsäure, 0,66 g (2,06 mmol) Quecksilber(II)-acetat und 0,12 g Hydrochinon in 75 ml Vinylacetat vorgelegt und unter Argon 20 min lang gerührt. Anschließend wurden als Katalysator 0,09 g (0,01 mol) p-Toluolsulfonsäure zugegeben, und das Reaktionsgemisch wurde 4 h lang rückflusserhitzt. Nach Abkühlen auf Raumtemperatur wurde die entstandene Lösung mit 200 ml Ethylacetat verdünnt und mit 150 ml 2 N NaOH extrahiert. Die organische Phase wurde über $Na_2SO_4$ getrocknet und die flüchtigen Komponenten anschließend am Rotationsverdampfer entfernt. Reinigung mittels Flash-Säulenchromatographie auf Kieselgel (PE:EE = 10:1) ergab 8,9 g (47 % d.Th.) einer farblosen Flüssigkeit.

$^1$H-NMR ($CDCl_3$), $\delta$ (ppm): 7,25 (2H, dd, J=14,07/6,25 Hz, $H_2C=C\underline{H}$-); 4,84 (2H, dd, J=14,07/1,56 Hz, -HC=C(H)<u>H</u>); 4,52 (2H, dd, J=6,25/1,56 Hz, -HC=C(<u>H</u>)H); 2,35 (4H, t, -CO-$CH_2$-); 1,62 (4H, $q_5$, -CO-$CH_2$-$C\underline{H}_2$-);1,29 (8H, s, -$CH_2$-).

<u>Synthesebeispiel 2: Synthese von Korksäuredivinylester (KVE)</u>

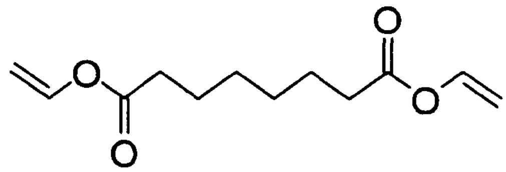

Durchführung analog Synthesebeispiel 1. Reinigung mittels Flash-Säulenchromatographie auf Kieselgel (PE:EE = 10:1) ergab 11,4 g (47 % d.Th.) einer farblosen Flüssigkeit.

$^1$H-NMR ($CDCl_3$), δ (ppm): 7,26 (2H, dd, J=13,84/6,21 Hz, $H_2C=C\underline{H}$-); 4,85 (2H, dd, J=13,84/1,47 Hz, -HC=C(H)$\underline{H}$); 4,54 (2H, dd, J=6,21/1,47 Hz, -HC=C($\underline{H}$)H); 2,37 (4H, t, -CO-$CH_2$-); 1,65 (4H, $q_5$, -CO-$CH_2$-C$\underline{H}_2$-);1,48-1,25 (4H, m, -$CH_2$-).

Synthesebeispiel 3: Synthese von N-Acetylphenylalaninvinylester (PAVE)

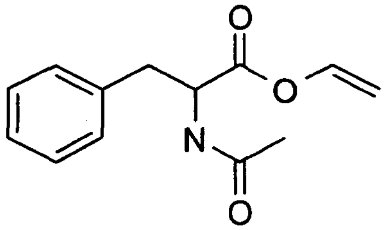

Lit.: M.I. Weinhouse, K.D. Janda, "A new methodology for the preparation of vinyl esters", Synthesis 1, 81-83 (1993).

a) Synthese von N-Acetylphenylalanin-2-(phenylseleno)ethylester

In einem 50-ml-Rundkolben wurden zu einer Lösung von 1,87 g (9,3 mmol) 2-(Phenylseleno)ethanol in 20 ml THF 2,3 g (18,8 mmol) 4-Dimethylaminopyridin, 2,55 g (13,3 mmol) 1-(3-Dimethylaminopropyl)-3-ethylcarbodiimid-hydrochlorid und 1,7 g (8,2 mmol) Phenylalanin zugegeben. Das Reaktionsgemisch wurde anschließend 12 h lang bei Raumtemperatur gerührt. Daraufhin wurde das Gemisch mit 50 ml Ethylacetat verdünnt und mit 50 ml 0,5 M HCl-Lösung extrahiert. Die organische Phase wurde über Natriumsulfat getrocknet, filtriert und das Lösungsmittel im Vakuum entfernt. Nach säulenchromatographischer Reinigung (PE:EE = 2:1) wurden 2,9 g (91 % d.Th., 98 % d.Lit.) einer gelblichen Flüssigkeit erhalten.

$^1$H-NMR ($CDCl_3$), ö (ppm): 7,3-7,1 (10H, m, Ar-H); 6,62 (1H, d, J=8,7 Hz, NH); 4,64 (1H, m, N-CH); 4,31 (2H, t, O-$CH_2$-); 3,25-3,01 (4H, m, -Ar-$CH_2$- + -Se-$CH_2$-); 1,97 (3H, s, -$CH_3$).

b) Synthese von N-Acetylphenylalaninvinylester (PAVE)

Zu einer Lösung von 2,6 g (6,66 mmol) N-Acetylphenylalanin-2-(phenylseleno)ethylester in 20 ml THF wurden bei 0 °C 8 ml einer 30%igen $H_2O_2$-Lösung langsam innerhalb von 10 min zugetropft und weitere 30 min lang bei 0 °C gerührt. Nachdem das Gemisch 12 h lang bei Raumtemperatur gerührt wurde, wurde mit 80 ml $CHCl_3$ verdünnt und mit 3 x 50 ml Wasser extrahiert. Die organischen Phasen wurden anschließend über Natriumsulfat getrocknet und das Lösungsmittel entfernt. Der Rückstand wurde in 70 ml Chloroform aufgenommen und 24 h lang rückflusserhitzt. Nach dem Abkühlen wurde das Lösungsmittel entfernt. Durch Flash-Säulenchromatographie (PE:EE = 3:1) wurden 1,3 g (84 % d.Th., 93 % d.Lit.) einer hellgelben Flüssigkeit erhalten.

$^1$H-NMR ($CDCl_3$), δ (ppm): 7,3-7,1 (6H, m, Ar-H + $H_2C$=CH-); 6,4 (1H, d, J=8,9 Hz, NH); 4,86 (1H, dd, J=13,67/1,51 Hz, -HC=CHH); 4,65 (1H, m, N-CH); 4,54 (1H, dd, J=6,18/1,51 Hz, -HC=CHH); 3,25-3,05 (2H, m, -$CH_2$-); 1,95 (3H, s,-$CH_3$).

Synthesebeispiel 4: Synthese von Diethylenglykol-bis[O-(O'-vinylmaleinoyl)polylactat (DVMPL)

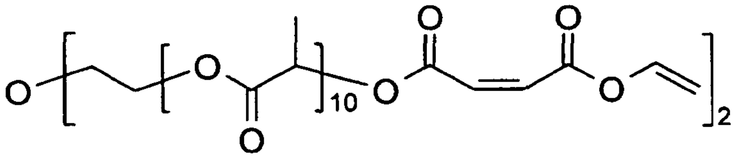

a) Synthese von Diethylenglykol-bispolylactat

0,74 g (6,9 mmol) Diethylenglykol wurden über Nacht mit $CaCl_2$ gerührt und anschließend filtriert. Der getrocknete Alkohol wurde dann gemeinsam mit dem 10 g (69 mmol) D,L-Lactid in einem Dreihalskolben vorgelegt und auf 130 °C erhitzt. Nachdem das D,L-Lactid geschmolzen war, wurden 94 mg (0,2 mmol) Sn-Octoat als Katalysator zugegeben, Vakuum angelegt und das Gemisch bei 130 °C 6 h lang gerührt. Nach dem Abkühlen wurde das Gemisch in wenig $CH_2Cl_2$ gelöst und mit kaltem Petrolether (PE) ausgefällt. Die überstehende Lösung wurde abdekantiert und der Rückstand ein weiteres Mal umgefällt. Es konnten 9 g (76% d.Th.) eines farblosen Feststoffs isoliert werden.
$^1$H-NMR ($CDCl_3$) $\delta$ (ppm): 5,13 (20H, m, CH-CO); 4,25 (4H, m, $CH_2$-O); 3,65 (4H, m, $CH_2$-O); 1,55 (60H, m, $CH_3$-C-O).

b) Synthese von Diethylenglykol-bis[(O-maleinoyl)polylactat]

8 g (5,2 mmol) Diethylenglykol-bispolylactat und 3,74 g (26,1 mmol) Maleinsäureanhydrid wurden in 100 ml Chloroform gelöst und 36 h lang auf 60 °C erhitzt. Nach Abkühlen auf Raumtemperatur wurden die flüchtigen Komponenten im Vakuum entfernt. Nach Umfällen aus Chloroform mit Petrolether wurden 8,4 g (98 % d.Th.) eines farblosen Feststoffs erhalten.
$^1$H-NMR ($CDCl_3$) $\delta$ (ppm): 6,59 (2H, m, =CH); 6,33 (2H, m, =CH); 5,12 (20H, m, CH-CO); 4,26 (4H, m, $CH_2$-O); 3,64 (4H, m, $CH_2$-O); 1,55 (60H, m, $CH_3$-C-O).

c) Synthese von Diethylenglykol-bis[O-(O'-phenylselenoethylmaleinoyl)polylactat]

Die Synthese erfolgte analog Synthesebeispiel 3a) mit 8,4 g (4,82 mmol) Diethylenglykol-bis[(O-maleinoyl)polylactat], 2,05 g (10,2 mmol) 2-(Phenylseleno)ethanol, 2,61 g (13,6 mmol) 1-(3-Dimethylaminopropyl)-3-ethylcarbodiimidhydrochlorid und 2,33 g (19,1 mmol) 4-Dimethylaminopyridin in 150 ml DMF. Nach Umfällen aus Chloroform mit PE wurden 9,8 g (96 % d.Th.) eines leicht gelben Feststoffs erhalten.
$^1$H-NMR ($CDCl_3$) $\delta$ (ppm): 7,52 (4H, m, Ar-H); 7,25 (4H, m, Ar-H); 6,51 (2H, d, =CH); 6,48 (2H, d, =CH); 5,12 (20H, m, CH-CO); 4,29 (8H, m, $CH_2$-O + $CH_2$-OCO); 3,64 (4H, m, $CH_2$-O); 3,07 (4H, t, Se-$CH_2$-); 1,53 (m, 60H, $CH_3$-C-O).

d) Synthese von Diethylenglykol-bis[O-(O'-vinylmaleinoyl)polylactat (DVMPL)

Die Synthese erfolgte analog Synthesebeispiel 3b) mit 9,8 g (4,64 mmol) Diethylenglykol-bis[O-(O'-phenylselenoethylmaleinoyl)polylactat] und 6 ml 30%iger $H_2O_2$-Lösung in 100 ml THF. Die Umsetzung zum Vinylester erfolgte in 150 ml Chloroform. Die Lösung wurde anschließend auf 50 ml eingeengt und das Produkt mit PE ausgefällt. Es wurden 7,8 g (95 % d.Th.) eines farblosen Feststoffs erhalten.
$^1$H-NMR ($CDCl_3$) $\delta$ (ppm): 7,26 (2H, m, $H_2$C=C<u>H</u>-); 6,61 (2H, m, =CH); 6,58 (2H, m, =CH); 5,12 (20H, m, CH-CO); 4,85 (2H, m, -HC=CH<u>H</u>); 4,54 (2H, m, -HC=C<u>H</u>H); 4,27 (4H, m, $CH_2$-O); 3,63 (4H, m, $CH_2$-O); 1,55 (60H, m, $CH_3$-C-O).

<u>Synthesebeispiel 5: Synthese von (2-Oxo-1,3-dioxolan-4-yl)methylmethacrylat (MC)</u>

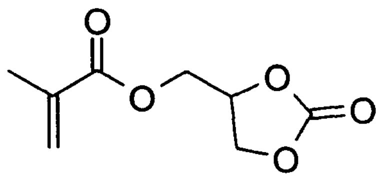

In einem 100-ml-Kolben wurden 10 ml (78,8 mmol) Triethylamin, 3 g (25,4 mmol) 4-Hydroxymethyl-1,3-dioxolan-2-on in 35 ml Methylenchlorid vorgelegt und mit Argon gespült. Bei 0 °C wurde dann Methacrylsäurechlorid langsam unter Rühren mittels einer Spritze zugetropft. Danach wurde das Reaktionsgemisch weitere 2 h lang bei Raumtemperatur gerührt. Das Gemisch wurde mit 100 ml 1 N HCl-Lösung und 100 ml Wasser extrahiert, die organischen Phasen über Natriumsulfat getrocknet, filtriert und das Lösungsmittel im Vakuum entfernt. Nach säulenchromatographischer Reinigung (PE:EE = 4:1) wurden 3,2 g (68 % d.Th.) einer farblosen Flüssigkeit erhalten.

$^1$H-NMR ($CDCl_3$), δ (ppm):6,13 (1H, s, H(H)C=); 5,64 (1H, s, H(H)C=); 5,05-4,87 (1H, m, -CH); 4,62-4,23 (4H, m, 2 x $-CH_2-$); 1,93 (3H, s, $-CH_3$).

Synthesebeispiel 6: Synthese von Trimerfettsäuretrivinylester (TFVE)

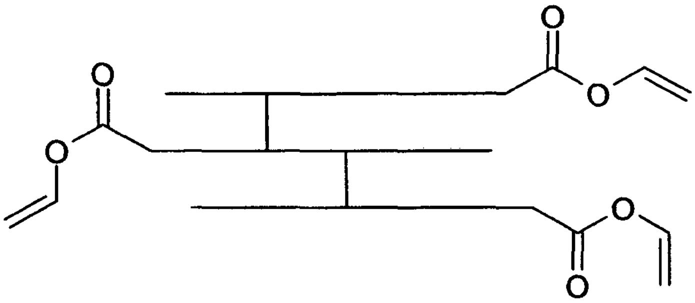

Herstellung analog Synthesebeispiel 1 aus Trimerfettsäure (Unidyme 60, Arizona Chemical). Bei säulenchromatographischer Reinigung auf Kieselgel (PE:EE = 5:1) wurden 38,2 g (75 % d. Th.) der Titelverbindung als gelbliche Flüssigkeit erhalten.

$R_f$ = 0,62 (PE:EE = 9:1)

$^1$H-NMR ($CDCl_3$), δ (ppm): 7,30 (3H, dd, J=6,26/14,09 Hz, $H_2C$=CH-O-CO); 4,88 (3H, dd, J=1,47/13,99 Hz, $H_2C$=CH-O-CO, cis); 4,55 (3H, dd, J=1,57/6,26 Hz, $H_2C$=CH-O-CO, trans); 2,39 (7,5H, t, $-CH_2$-COO-); 1,56-0,86 (99,2H, bm, Alkyl-H).

IR (ATR, Dünnfilm): 2927, 2853, 1750, 1650, 1462, 1141, 954, 870 $cm^{-1}$.

Synthesebeispiel 7: Synthese von ω,ω'-3,6,9-Trioxaundecandisäuredivinylester (TUVE)

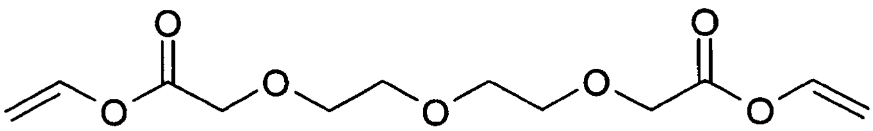

In einem 250-ml-Dreihalskolben wurden 2,94 g (13,4 mmol) 3,6,9-Trioxaundecandisäure, 0,46 g (0,7 mmol) Palladium(II)-acetat und 0,08 g Kaliumhydroxid in 60 ml Vinylacetat vorgelegt und unter Argon 70 h lang bei 50 °C gerührt. Nach dem Abkühlen auf Raumtemperatur wurde die erhaltene Lösung mit 200 ml Ethylacetat verdünnt und zweimal mit 100 ml Wasser extrahiert. Die organische Phase wurde über $Na_2SO_4$ getrocknet, und die flüchtigen Komponenten wurden anschließend am Rotationsverdampfer entfernt. Reinigung mittels Flash-Säulenchromatographie auf Kieselgel (PE:EE = 1:1) ergab 0,95 g (26 % d.Th.) einer gelben Flüssigkeit.

$R_f$ = 0,67 (PE/EA 1:1)

$^1$H-NMR ($CDCl_3$), δ (ppm): 7,29 (2H, dd, J=6,26/13,89 Hz, $H_2C$=CH-O-CO); 4,92 (2H, dd, J=1,76/13,89 Hz, $H_2C$=CH-O-CO, cis); 4,63 (3H, dd, J=1,76/6,26 Hz, $H_2C$=CH-O-CO, trans); 4,24 (4H, s, $-O-CH_2$-COO-); 3,80-3,64 (4H, m, $-O-CH_2-CH_2-O-$).

IR (ATR, Dünnfilm): 2932, 2882, 1768, 1649, 1240, 1181, 1112, 949, 875 $cm^{-1}$.

Synthesebeispiel 8: Synthese von 1,2-Ethylenglykol-bis(vinylcarbonat) (EGDVC)

1,2-Ethandiyl-bis(vinylcarbonat), Kohlensäure-vinyl-2-(vinyloxycarbonyloxy)ethylester

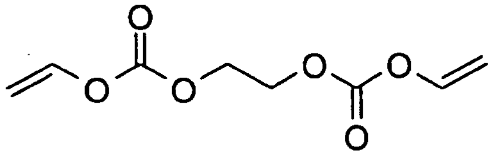

In einem 100-ml-Einhalskolben wurden 1,5 g (24,2 mmol) Ethylenglykol und 13,3 g (167 mmol) Pyridin in 50 ml Dichlormethan vorgelegt. Anschließend wurde das Reaktionsgemisch mit einem Eisbad auf 0 °C abgekühlt, und binnen 5 min wurden unter Rühren 5,56 g (52,2 mmol) Chlorameisensäurevinylester mittels einer Spritze unter Argonatmosphäre zugetropft. Nach vollständiger Zugabe wurde weitere 5 min lang bei 0 °C gerührt. Das Eisbad wurde entfernt und das Reaktionsgemisch 1 h lang bei Raumtemperatur gerührt. Das Reaktionsgemisch wurde anschließend mit 100 ml Dichlormethan verdünnt und mit 150 ml 1 N Salzsäure extrahiert. Die organische Phase wurde danach mit 100 ml gesättigter Natriumchlorid-Lösung gewaschen und über Natriumsulfat getrocknet. Das Lösungsmittel wurde in Gegenwart einer Spatelspitze Hydrochinon abdestilliert. Nach Reinigung mittels Flash-Säulenchromatographie (PE:EE = 5:1) wurden 4,1 g (84 % d. Th.) der Titelverbindung als farblose Flüssigkeit erhalten.

$R_f$ = 0,44 (PE:EE =5:1)

$^1$H-NMR ($CDCl_3$), $\delta$ (ppm): 7,06 (2H, dd, J=6,18/13,75 Hz, $H_2C$=CH-O-CO); 4,92 (2H, dd, J=2,37/13,75 Hz, $H_2C$=CH-O-CO); 4,63 (2H, dd, J=2,37/6,18 Hz, $H_2C$=CH-O-CO, trans); 4,43 (4H, s, -$CH_2$-).

Synthesebeispiel 9: Synthese von 1,4-Butandiol-bis(vinylcarbonat) (BDDVC)

Butan-1,4-diyl-bis(vinylcarbonat), Kohlensäure-vinyl-4-(vinyloxycarbonyloxy)butylester

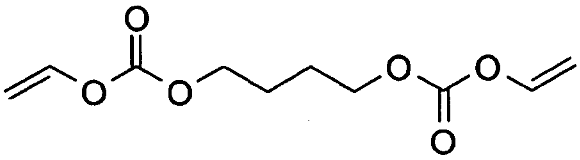

Herstellung analog Synthesebeispiel 8 aus 1,4-Butandiol und Chlorameisensäurevinylester. Bei säulenchromatographischer Reinigung auf Kieselgel (PE:EE = 3:1) wurden 3,6 g (94 % d. Th.) der Titelverbindung als farblose Flüssigkeit erhalten.

$R_f$ = 0,77 (PE:EE = 3:1)

$^1$H-NMR ($CDCl_3$), $\delta$ (ppm): 7,07 (2H, dd, J=6,16/13,78 Hz, $H_2C$=C$\underline{H}$-O-CO); 4,91 (2H, dd, J=2,06/13,78 Hz, $\underline{H_2}C$=CH-O-CO, cis); 4,57 (2H, dd, J=2,05/6,17 Hz, $\underline{H_2}C$=CH-O-CO, trans); 4,23 (4H, t, OC-O-C$\underline{H_2}$-$CH_2$-); 1,81 (4H, m, OC-O-$CH_2$-C$\underline{H_2}$-).

Elementaranalyse ($C_{10}H_{14}O_6$): ber. C: 52,17, H: 6,13;
gef. C: 51,78, H: 6,26.

Synthesebeispiel 10: Synthese von 1,6-Hexandiol-bis(vinylcarbonat) (HDDVC)

Hexan-1,6-diyl-bis(vinylcarbonat), Kohlensäure-vinyl-6-(vinyloxycarbonyloxy)hexylester

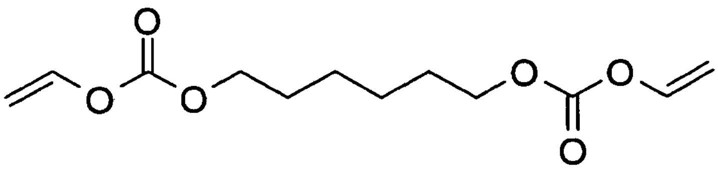

Herstellung analog Synthesebeispiel 8 aus 1,6-Hexandiol und Chlorameisensäurevinylester. Bei säulenchromatographischer Reinigung auf Kieselgel (PE:EE = 5:1) wurden 5,5 g (84 % d. Th.) der Titelverbindung als farblose Flüssigkeit erhalten.

$R_f$ = 0,53 (PE:EE = 5:1) $^1$H-NMR ($CDCl_3$), δ (ppm): 7,07 (2H, dd, J=6,16/13,78 Hz, $H_2C{=}C\underline{H}$-O-CO); 4,91 (2H, dd, J=2,06/13,78 Hz, $\underline{H_2}C{=}CH$-O-CO, cis); 4,57 (2H, dd, J=2,05/6,17 Hz, $\underline{H_2}C{=}CH$-O-CO, trans); 4,18 (4H, t, OC-O-$CH_2$-$C\underline{H_2}$-); 1,70 (4H, m, OC-O-$CH_2$-$C\underline{H_2}$-); 1,41 (4H, m, -$CH_2$-$C\underline{H_2}$-$C\underline{H_2}$-$CH_2$-).

Synthesebeispiel 11: Synthese von Diethylenglykol-bis(vinylcarbonat) (DEGDVC)

3-Oxapentan-1,5-diyl-bis(vinylcarbonat), Kohlensäure-vinyl-2-[2-(vinyloxycarbonyloxy)ethoxy]ethyl-ester

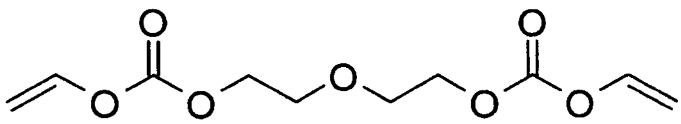

Herstellung analog Synthesebeispiel 8 aus Diethylenglykol und Chlorameisensäurevinylester. Bei säulenchromatographischer Reinigung auf Kieselgel (PE:EE = 5:1) wurden 3,3 g (95 % d. Th.) der Titelverbindung als farblose Flüssigkeit erhalten.

$R_f$ = 0,42 (PE:EE = 3:1)

$^1$H-NMR ($CDCl_3$), δ (ppm): 7,03 (2H, dd, J=6,26/13,88 Hz, $H_2C{=}CH$-O-CO); 4,87 (2H, dd, J=2,06/13,78 Hz, $\underline{H_2}C{=}CH$-O-CO, cis); 4,54 (2H, dd, J=1,96/6,26 Hz, $\underline{H_2}C{=}CH$-O-CO, trans); 4,31 (4H, t, (OC-O-C-$\underline{H_2}$-$CH_2$)$_2$-O); 3,71 (4H, t, (OC-O-$CH_2$-$C\underline{H_2}$)$_2$-O).

Synthesebeispiel 12: Synthese von Polyethylenglykol(400)-bisvinylcarbonat (PEGDVC)

Kohlensäure-vinyl-2-[ω-(vinyloxycarbonyloxy)polyoxyethylen(400)oxy]ethyl-ester

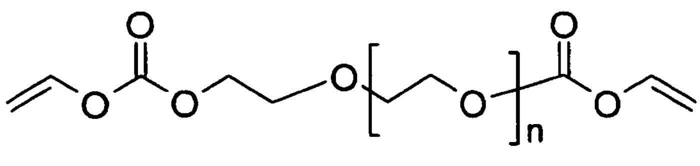

Herstellung analog Synthesebeispiel 8 aus Polyethylenglykol-400 und Chlorameisensäurevinylester. Bei säulenchromatographischer Reinigung auf Kieselgel (PE:EE = 1:2) wurden 2,0 g (93 % d. Th.) der Titelverbindung als farblose Flüssigkeit erhalten.

$^1$H-NMR ($CDCl_3$), δ (ppm): 7,07 (2H, dd, J=6,26/13,79 Hz, $H_2C{=}C\underline{H}$-O-CO); 4,91 (2H, dd, J=1,96/13,79 Hz, $\underline{H_2}C{=}CH$-O-CO, cis); 4,57 (2H, dd, J=1,96/6,26 Hz, $\underline{H_2}C{=}CH$-O-CO, trans); 4,34 (4H, t, CO-O-$C\underline{H_2}$-); 3,79-3,56 (26H, m, -$C\underline{H_2}$-O-).

IR (ATR, Dünnfilm): 1758 (C=O), 1650 (C=C), 1241, 1152 $cm^{-1}$.

Synthesebeispiel 13: Synthese von Diethylenglykol-bis[O-(O'-vinyloxycarbonyl)polylactat] (DEG(PLAVC)$_2$)

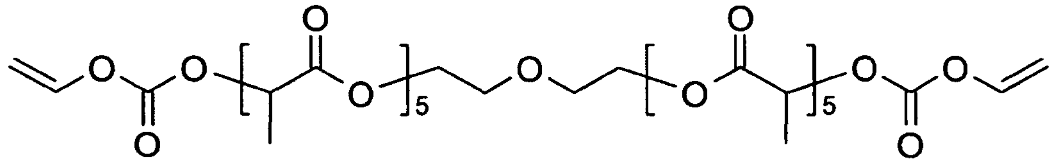

Herstellung analog Synthesebeispiel 8 aus Diethylenglykol-bispolylactat und Chlorameisensäurevinylester. Die Reinigung erfolgte durch Aufnehmen in $CHCl_3$ und Ausölen mit kaltem PE. Es wurden 4,1 g (94 % d. Th.) der Titelverbindung als farbloses, hochviskoses Öl erhalten.

$^1$H-NMR ($CDCl_3$), $\delta$ (ppm): 7,04 (2H, dd, J=6,06/13,68 Hz, =CH-O-CO); 5,16 (10H, m, O-C$\underline{H}$($CH_3$)-COO); 4,96 (2H, dd, J=1,94/13,88 Hz, $\underline{H}_2$C=CH-O-CO, cis); 4,61 (2H, dd, J=1,94/6,06 Hz, $\underline{H}_2$C=CH-O-CO, trans); 4,40-4,18 (4H, m, OC-O-C$\underline{H}_2$-$CH_2$-O); 3,72-3,60 (4H, m, OC-O-$CH_2$-C$\underline{H}_2$-O); 1,70-1,35 (30H, m, O-CH(C$\underline{H}_3$)-COO).

IR (ATR, Dünnfilm): 1750 (C=O), 1652 (C=C), 1263, 1188, 1085 $cm^{-1}$.

Synthesebeispiel 14: Synthese von 2-Cyanoethylvinylcarbonat (CEVC)

Kohlensäure-2-cyanoethyl-vinyl-ester

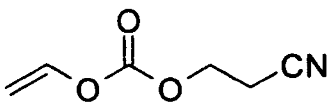

Herstellung analog Synthesebeispiel 8 aus 2-Cyanoethanol (Hydroxypropionitril) und Chlorameisensäurevinylester. Bei säulenchromatographischer Reinigung auf Kieselgel (PE:EE = 3:1) wurden 2,8 g (92 % d. Th.) der Titelverbindung als farblose Flüssigkeit erhalten.

$R_f$ = 0,54 (PE:EE = 3:1)

$^1$H-NMR ($CDCl_3$), $\delta$ (ppm): 7,04 (1H, dd, J=6,16/13,78 Hz, $H_2$C=CH-O-CO); 4,96 (1H, dd, J=2,24/13,78 Hz, $H_2$C=CH-O-CO, cis); 4,64 (1H, dd, J=2,14/6,06 Hz, $\underline{H}_2$C=CH-O-CO, trans); 4,39 (2H, t, OC-O-$CH_2$); 2,78 (2H, t, $CH_2$-CN).

Elementaranalyse ($C_6H_7NO_3$): ber. C: 51,07, H: 5,00, N: 9,92;
gef. C: 51,21, H: 4,98, N: 9,73.

Synthesebeispiel 15: Synthese von Glycerin-tris(vinylcarbonat) (GTVC)

Propan-1,2,3-triyl-tris(vinylcarbonat), Kohlensäure-2,3-bis(vinyloxycarbonyloxy)-propyl-vinyl-ester

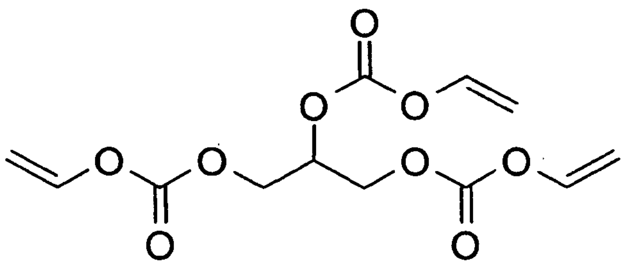

Herstellung analog Synthesebeispiel 8 aus Glycerin und Chlorameisensäurevinylester. Bei säulenchromatographischer Reinigung auf Kieselgel (PE:EE = 5:1) wurden 0,8 g (75 % d. Th.) der Titelverbindung als farblose Flüssigkeit erhalten.

$R_f$ = 0,64 (PE:EE = 3:1)

$^1$H-NMR ($CDCl_3$), $\delta$ (ppm): 7,04 (3H, dd, J=6,16/13,78 Hz, $H_2C{=}CH{-}O{-}CO$); 5,21 (1H, m, $(OC{-}O{-}H_2C)_2C\underline{H}{-}O{-}CO$); 4,94 (3H, dd, J=2,67/11,73 Hz, $\underline{H_2}C{=}CH{-}O{-}CO$, cis); 4,62 (3H, dd, J=2,34/6,24 Hz, $\underline{H_2}C{=}CH{-}O{-}CO$, trans); 4,45 (4H, m, $(OC{-}O{-}\underline{H_2}{-}C)_2{-}CH{-}O{-}CO$).

<u>Synthesebeispiel 16: Synthese von Ethylvinylcarbonat (EVC)</u>

Kohlensäure-ethyl-vinyl-ester

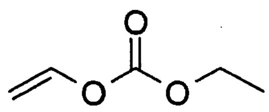

Herstellung analog Synthesebeispiel 8 aus Ethanol und Chlorameisensäurevinylester. Bei säulenchromatographischer Reinigung auf Kieselgel (PE:EE = 5:1) wurden 1,3 g (83 % d. Th.) der Titelverbindung als farblose Flüssigkeit erhalten.

$R_f$ = 0,58 (PE:EE = 3:1)

$^1$H-NMR ($CDCl_3$), $\delta$ (ppm): 7,06 (1H, dd, J=6,26/13,88 Hz, $H_2C{=}C\underline{H}{-}O{-}CO$); 4,88 (1H, dd, J=1,96/13,88 Hz, $\underline{H_2}C{=}CH{-}O{-}CO$, cis); 4,54 (1H, dd, J=1,96/6,06 Hz, $\underline{H_2}C{=}CH{-}O{-}CO$, trans); 4,24 (2H, q, $OC{-}O{-}C\underline{H_2}{-}CH_3$); 1,31 (3H, t, $OC{-}O{-}CH_2{-}C\underline{H_3}$).

<u>Synthesebeispiel 17: Synthese von Ricinusöl-tris(vinylcarbonat) (RiTVC)</u>

Hauptsächlich (zu etwa 80 %): Propan-1,2,3-triyl-tris[(R)-(Z)-12-vinyloxycarbonyloxy-9-octadecenoat]

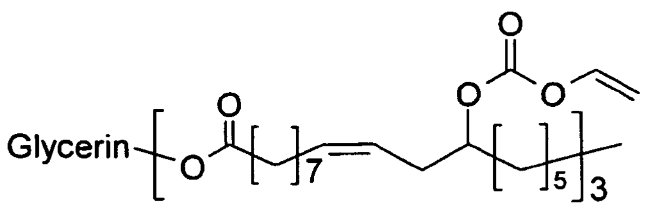

Herstellung analog Synthesebeispiel 8 aus Ricinusöl und Chlorameisensäurevinylester. Bei säulenchromatographischer Reinigung auf Kieselgel (PE:EE = 5:1) wurden 1,5 g (53 % d. Th.) einer farblosen, viskosen Flüssigkeit erhalten.

$^1$H-NMR ($CDCl_3$), $\delta$ (ppm): 7,08 (3H, dd, J=6,26/13,88 Hz, $H_2C{=}C\underline{H}{-}O{-}CO$); 5,55-5,22 (8H, m, $-CH_2{-}C\underline{H}{=}C\underline{H}{-}CH_2$ und $CH_2)_2{-}C\underline{H}{-}O{-}CO$); 4,88 (3H, dd, J=1,76/13,88 Hz, $\underline{H_2}C{=}CH{-}O{-}CO$, cis); 4,75 (3H, quin, $CH_2{-}C\underline{H}O{-}CH_2$); 4,55 (3H, dd, J=1,76/6,06 Hz, $\underline{H_2}C{=}CH{-}O{-}CO$, trans); 4,23 (2H, dd, $CO{-}O{-}CH_2$); 4,19 (2H, dd, $CO{-}O{-}CH_2$); 2,45-2,22 (12H, m, $OOC{-}C\underline{H_2}{-}CH_2$ und $HC{=}CH{-}C\underline{H_2}{-}COH$); 2,12-1,95 (6H, m); 1,72-1,10 (60H, m); 1,02-0,80 (9H, m).

IR (ATR, Dünnfilm): 1751 (C=O), 1650 (C=C), 1252, 1158 $cm^{-1}$.

<u>Synthesebeispiel 18: Synthese von hydriertem Ricinusöl-tris(vinylcarbonat) (HRiTVC)</u>

Hauptsächlich (zu etwa 80 %): Propan-1,2,3-triyl-tris[(R)-(Z)-12-vinyloxycarbonyloxy-octadecanoat]

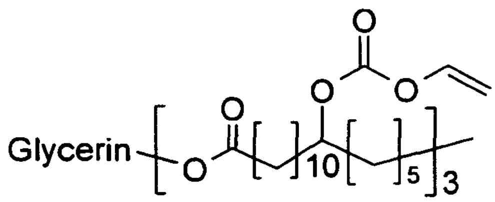

Herstellung analog Synthesebeispiel 8 aus hydriertem Ricinusöl (Loxiol G 15, Oleo Chemicals) und Chlorameisensäurevinylester. Bei säulenchromatographischer Reinigung auf Kieselgel (PE:EE = 5:1) wurden 0,77 g (77 % d. Th.) einer farblosen, viskosen Flüssigkeit erhalten.

$^1$H-NMR ($CDCl_3$), δ (ppm): 7,10 (3H, dd, J=6,16/13,79 Hz, $H_2C{=}C\underline{H}$-O-CO); 5,39-5,20 (8H, m, $-CH_2-C\underline{H}{=}C\underline{H}-CH_2$ [6H] und $(CH_2)_2-C\underline{H}$-O-CO [2H]); 4,90 (3H, dd, J=1,86/ 13,79 Hz, $\underline{H_2}C{=}CH$-O-CO, cis); 4,75 (1H, m, $CH_2-C\underline{H}O-CH_2$); 4,55 (3H, dd, J=1,86/ 6,16 Hz, $\underline{H_2}C{=}CH$-O-CO, trans); 4,29 (2H, dd, OCO-$C\underline{H_2}$-COH); 4,14 (2H, dd, OCO-$C\underline{H_2}$-COH); 2,45-2,22 (12H, m, OOC-$CH_2$-$C\underline{H_2}$ [6H] und HC=CH-$C\underline{H_2}$-COH [6H]); 2,31 (6H, t); 1,70-1,50 (18H, m); 1,50-1,20 (81H, m); 1,08-0,82 (14H, m).

IR (ATR, Dünnfilm): 2932, 2858, 1753, 1462, 1250, 1156, 949, 865 $cm^{-1}$.

Synthesebeispiel 19: Synthese von N,N'-Dimethyl-1,2-ethylendiamin-bis(vinylcarbamat) (DMEDDVCA)

N,N'-Dimethyl-N,N'-ethan-1,2-diyl-bis(carbaminsäure), N-Methyl-N-2-[N'-methyl-N'-(vinyloxycarbonyl)amino]ethyl-carbaminsäurevinylester

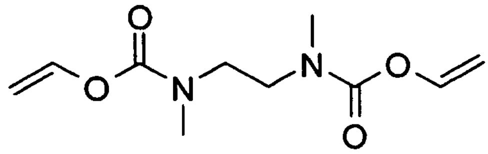

Herstellung analog Synthesebeispiel 8 aus N,N'-Dimethyl-1,2-ethylendiamin und Chlorameisensäurevinylester. Bei säulenchromatographischer Reinigung auf Kieselgel (PE:EE = 1:1) wurden 3,1 g (96 % d. Th.) der Titelverbindung als farblose Flüssigkeit erhalten.

$R_f$ = 0,47 (PE:EE = 1:1)

$^1$H-NMR ($CDCl_3$), δ (ppm): 7,17 (2H, m, $H_2C{=}CH$-O-CO); 4,75 (2H, m, $\underline{H_2}C{=}CH$-O-CO, cis); 4,42 (2H, m, $\underline{H_2}C{=}CH$-O-CO, trans); 3,47 (4H, s, N-$CH_2$); 2,98 (6H, s, N-$CH_3$).

Elementaranalyse ($C_{10}H_{16}N_2O_4$): ber. C: 52,62, H: 7,07, N: 12,27;
gef. C: 52,34, H: 6,99, N: 12,10.

Synthesebeispiel 20: Synthese von Piperazin-bis(vinylcarbamat) (PDVCA)

Diethylendiamin-bis(vinylcarbamat), N,N'-Bis(vinyloxycarbonyl)hexahydro-1,4-diazin

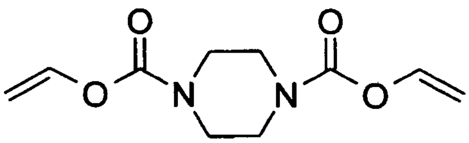

Herstellung analog Synthesebeispiel 8 aus Piperidin und Chlorameisensäurevinylester. Bei säulenchromatographischer Reinigung auf Kieselgel (PE:EE = 1:1) wurden 1,2 g (91 % d. Th.) der Titelverbindung als farblose Flüssigkeit erhalten.

$R_f$ = 0,70 (PE:EE = 1:1) $^1$H-NMR ($CDCl_3$), δ (ppm): 7,21 (2H, dd, J=6,36/13,98 Hz, $H_2C$=CH-O-CO); 4,81 (2H, dd, J=1,66/11,73 Hz, $H_2C$=CH-O-CO, cis); 4,50 (2H, dd, J=1,76/6,26, $H_2C$=CH-O-CO, trans); 3,56 (8H, s, $CH_2$-$CH_2$).

Synthesebeispiel 21: Synthese von 3,3'-Ethylendioxy-bis(propylamin)divinylcarbamat (Jeffamin-bis(vinylcarbamat), JAVM)

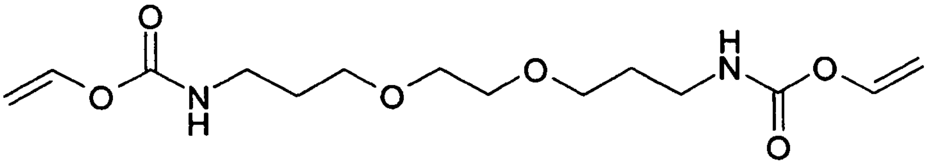

Herstellung analog Synthesebeispiel 8 aus 3,3'-Ethylendioxy-bis(propylamin) und Chlorameisensäurevinylester. Bei säulenchromatographischer Reinigung auf Kieselgel (PE:EE = 1:1) wurden 1,03 g (72 % d. Th.) der Titelverbindung als farblose Flüssigkeit erhalten.

$R_f$ = 0,43 (PE:EE = 1:1)

$^1$H-NMR ($CDCl_3$), δ (ppm): 7,20 (2H, dd, J=6,36/13,99 Hz, $H_2C$=CH-O-CO); 5,93-5,73 (0,6H, bs, -OC-NH-$CH_2$-); 5,65-5,39 (1,4H, bs, -OC-NH-$CH_2$-); 4,69 (2H, dd, J=1,37/ 14,09 Hz, $H_2C$=CH-O-CO, cis); 4,40 (2H, dd, J=1,08/6,36 Hz, $H_2C$=CH-O-CO, trans); 3,66-3,46 (8H, m, -$CH_2$-O-$CH_2$-$CH_2$-O-$CH_2$-); 3,35 (4H, q, -$CH_2$-$CH_2$-NH-CO-); 1,82 (4H, quin, -O-$CH_2$-$CH_2$-$CH_2$-NH-).

IR (ATR, Dünnfilm): 3331, 2932, 2872, 1718, 1649, 1526, 1240, 1166, 1101, 954, 860 $cm^{-1}$.

Synthesebeispiel 22: Synthese von Bis[ω-aminopolyethylenglykol(500)]amin-trivinyl-carbamat (EAVM)

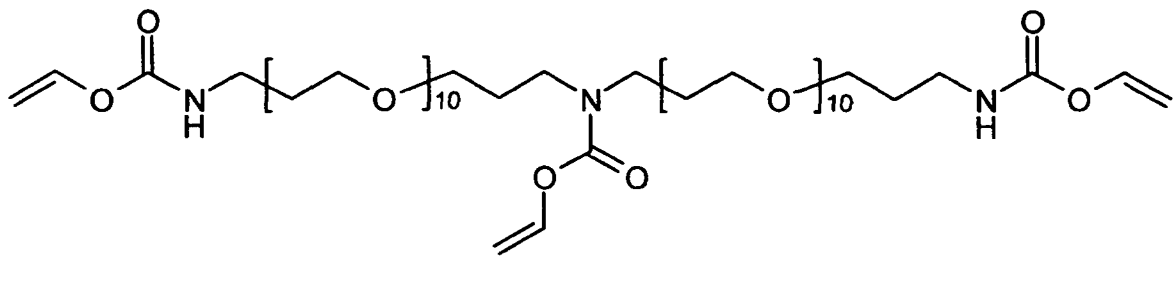

Herstellung analog Synthesebeispiel 8 aus Bis[ω-aminopolyethylenglykol(500))amin (Jeffamine EDH-176, Huntsman) und Chlorameisensäurevinylester. Bei säulenchromatographischer Reinigung auf Kieselgel (PE:EE = 1:1) wurden 2,02 g (86 % d. Th.) der Titelverbindung als farblose Flüssigkeit erhalten.

$^1$H-NMR ($CDCl_3$), δ (ppm): 7,14 (2H, dd, J=6,36/13,99 Hz, $H_2C$=CH-O-CO-NH); 7,13 (1H, dd, J=6,36/13,99 Hz, $H_2C$=CH-O-CO-N($CH_2$)$_2$); 5,82-5,62 (2H, bs, -OC-NH-$CH_2$-); 4,70 (1H, dd, J=1,47/13,99Hz, $H_2C$=CH-O-CO-N($CH_2$)$_2$, cis); 4,65 (2H, dd, J=1,27/13,99 Hz, $H_2C$=CH-O-CO-NH, cis); 4,39 (1H, dd, J=1,37/6,26Hz, $H_2C$=CH-O-CO-N($CH_2$)$_2$, trans); 4,35 (2H, dd, J=1,37/6,26Hz, $H_2C$=CH-O-CO-NH, trans); 3,72-3,40 (92H, m, -$CH_2$-O-$CH_2$-$CH_2$-O-$CH_2$-); 3,37-3,30 (4H, m, -$CH_2$-$CH_2$-NH-CO-).

IR (ATR, Dünnfilm): 3336, 2872, 1743, 1718, 1649, 1526, 1245, 1101, 949, 860 $cm^{-1}$.

Synthesebeispiel 23: Synthese von Sarkosinmethylester-vinylcarbamat (SMEVCA)

N-Methyl-N-(vinyloxycarbonyl)glycin-methylester, N-(Methoxycarbonylmethyl)-N-methyl-carbaminsäurevinylester

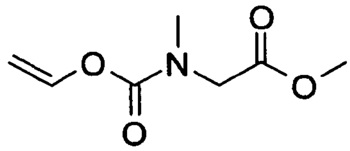

Herstellung analog Synthesebeispiel 8 aus Sarkosin und Chlorameisensäurevinylester. Bei säulenchromatographischer Reinigung auf Kieselgel (PE:EE = 1:1) wurden 1,9 g (96 % d. Th.) der Titelverbindung als farblose Flüssigkeit erhalten.
$R_f$ = 0,57 (PE:EE = 1:1)
$^1$H-NMR ($CDCl_3$), δ (ppm): 7,12 (1H, m, $H_2C$=CH-O-CO); 4,76 (1H, m, $H_2C$=CH-O-CO, cis); 4,44 (1H, m, $\underline{H}_2C$=CH-O-CO, trans); 4,03 (2H, s, N-$CH_2$-COO); 4,03 (3H, s, CO-O-$CH_3$); 2,99 (3H, s, N-$CH_3$).

Elementaranalyse ($C_7H_{11}NO_4$): ber. C: 48,55, H: 6,40, N: 8,09;
gef. C: 48,51, H: 6,56, N: 8,02.

Synthesebeispiel 24: Synthese von N,O-Bis(vinyloxycarbonyl)-N-methylhydroxylamin (MHADVC)

N-Methyl-N-(vinyloxycarbonyloxy)-carbaminsäurevinylester

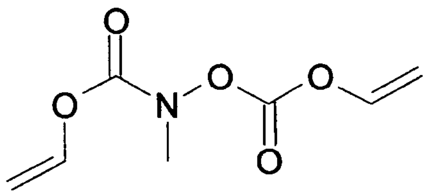

Durchführung analog Synthesebeispiel 8 aus N-Methylhydroxylamin und Chlorameisensäurevinylester. Bei säulenchromatographischer Reinigung auf Kieselgel (PE:EE = 6:1) wurden 1,4 g (86 % d. Th.) der Titelverbindung als farblose Flüssigkeit erhalten.
$R_f$ = 0,36 (PE:EE = 6:1)
$^1$H-NMR ($CDCl_3$), δ (ppm): 7,17-7,00 (2H, m, $CH_2$=C$\underline{H}$-O-CO); 5,09-5,1 (1H, dd, J=2,4/13,7 Hz, C$\underline{H}_2$=CH-O-CO-N, cis); 4,94-4,86 (1H, dd, J=2,0/13,9 Hz, C$\underline{H}_2$=CH-O-CO-O, cis); 4,75-4,71 (1H, dd, J=2,6/6,07 Hz, C$\underline{H}_2$=CH-O-CO-N. trans); 4,62-4,58 (1H, dd, J=2,1/6,2 Hz, C$\underline{H}_2$=CH-O-CO-O, trans); 3,75 (3H, s, -N$CH_3$).

Elementaranalyse ($C_7H_9NO_5$): ber. C: 44,92, H: 4,85, N: 7,48;
gef. C: 44,54, H: 5,06, N: 7,24.

Synthesebeispiel 25: Synthese von N-Methoxyvinylcarbamat (MVCA)

N-Methoxy-carbaminsäurevinylester

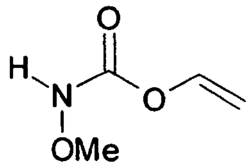

Durchführung analog Synthesebeispiel 8 aus O-Methylhydroxylamin und Chlorameisensäurevinylester. Bei säulenchromatographischer Reinigung auf Kieselgel (PE:EE = 8:1) wurden 0,9 g (79 % d. Th.) der Titelverbindung als farblose Flüssigkeit erhalten.

$R_f$ = 0,18 (PE:EE = 8:1)

$^1$H-NMR ($CDCl_3$), δ (ppm): 7,22-7,112 (1H, dd, J=6,1/13,7 Hz, $H_2C$=CH-O-CO); 4,88-4,80 (1H, dd, J=2,0/13,7 Hz, $\underline{H}_2C$=CH-O-CO, cis); 4,57-4,53 (1H, dd, J=1,9/6,3 Hz, $H_2C$=CH-O-CO, trans); 3,76 (3H, s, O-$CH_3$).

| | |
|---|---|
| Elementaranalyse ($C_4H_7NO_3$): | ber. C: 41,03, H: 6,03, N: 11,96; |
| | gef. C: 41,25, H: 6,16, N: 11,74. |

Synthesebeispiel 26: Synthese von N-Acryloyl-N-methoxyvinylcarbamat (AMVCA)

N-Methoxy-N-propenoyl-carbaminsäurevinylester

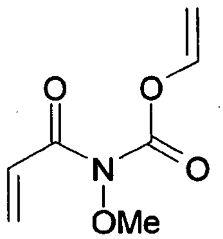

Durchführung analog Synthesebeispiel 8 aus N-Methoxyacrylamid und Chlorameisensäurevinylester. Bei säulenchromatographischer Reinigung auf Kieselgel (PE:EE = 9:1) wurden 1,6 g (91 % d. Th.) der Titelverbindung als farblose Flüssigkeit erhalten.

$R_f$ = 0,34 (PE:EE = 9:1)

$^1$H-NMR ($CDCl_3$), δ (ppm): 7,12-7,02 (1H, dd, J=6,3/13,8 Hz, $CH_2$=C$\underline{H}$-O-CO); 6,29-6,21 (1H, dd, J=11,0/17,5 Hz, $CH_2$=C$\underline{H}$-CO-N); 5,80-5,71 (1H, dd, J=0,5/17,5 Hz, C$\underline{H}_2$=CH-CO-N, cis); 5,58-5,25 (1H, dd, J=0,5/11,3 Hz, C$\underline{H}_2$=CH-CO-N, trans); 5,07-4,99 (1H, dd, J=2,3/13,7 Hz, C$\underline{H}_2$=CH-O-CO, cis); 4,71-4,67 (1H, dd, J=2,3/6,1 Hz, C$\underline{H}_2$=CH-O-CO, trans); 3,90 (3H, s, -$OCH_3$).

| | |
|---|---|
| Elementaranalyse ($C_7H_9NO_3$): | ber. C: 49,12, H: 5,30, N: 8,18; |
| | gef. C: 49,08, H: 5,38, N: 8,22. |

Synthesebeispiel 27: Synthese von Vinyloxycarbonylphosphonsäurediethylester (VCPDE)

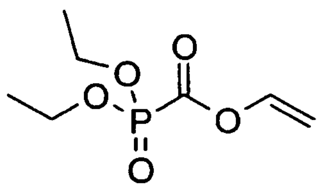

2,0 g (19 mmol) Chlorameisensäurevinylester wurde in einem 50-ml-Zweihalskolben vorgelegt, und bei 0°C wurden unter Rühren 3,13 g (19 mmol) Triethylphosphit langsam zugetropft. Nach vollständiger Zugabe wurde das Reaktionsgemisch 2 h lang bei Raumtemperatur weitergerührt. Zur vollständigen Entfernung des bei der Reaktion entstehenden Ethylchlorids wurde die Lösung 30 min lang auf 40 °C erwärmt. Bei Reinigung mittels Vakuumdestillation wurden 2,5 g (64 % d. Th.) der Titelverbindung als farblose Flüssigkeit erhalten.

Kp.: 125-128 °C/8 mbar

$R_f$= 0,35 (PE:EE = 1:1)

$^1$H-NMR ($CDCl_3$), $\delta$ (ppm): 7,01-6,99 (1H. dd, J=0,7/6,3 Hz, $H_2C=C\underline{H}$-O-CO); 4,88-4,79 (1H, m, $\underline{H_2}C$=CH-O-CO, cis); 4,55-4,48 (1H, m, $\underline{H_2}C$=CH-O-CO, trans); 4,12-3,97 (4H, m, O-$CH_2$); 1,17-1,09 (6H, m, -$CH_3$).

Elementaranalyse ($C_7H_{13}O_5P$): ber. C: 40,39, H: 6,30, P: 14,88;
gef. C: 40,60, H: 6,24, P: 14,71.

Synthesebeispiel 28: Synthese von 2-(Diethoxyphosphoryloxy)ethylamin-vinylcarbamat (EPEVC)

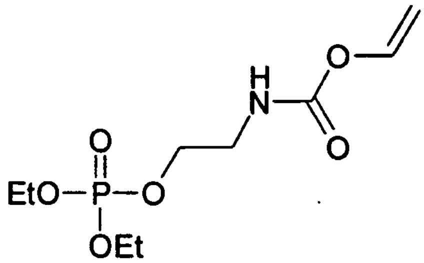

0,81 ml Triethylamin (5,8 mmol) wurden in 10 ml absolutem THF vorgelegt und 0,76 g 2-Hydroxyvinylcarbamat (5,8 mmol) zugesetzt. Anschließend wurde die Reaktionslösung auf -78°C abgekühlt, und 0,83 ml Diethylchlorphosphonit (5,8 mmol) in 4 ml THF wurden zugetropft. Nach Beendingung des Zutropfens wurde das Reaktionsgemisch 12 h lang bei Raumtemperatur gerührt. Ein weißer Feststoff wurde abfiltriert, und der Rückstand wurde mit 5%iger wässriger Natriumhydrogencarbonat-Lösung (3 x 10 ml) gewaschen. Anschließend wurde die organische Phase über Natriumsulfat getrocknet. Nach Abfiltrieren des Trocknungsmittels wurde das Lösungsmittel am Rotationsverdampfer entfernt. Das Rohprodukt wurde säulenchromatographisch (PE:EE = 1:5) auf Kieselgel gereinigt. Es wurden 0,39 g (25 % d. Th.) der Titelverbindung als farbloses Öl erhalten.

$R_f$ = 0,28 (PE:EE = 1:5)

$^1$H-NMR ($CDCl_3$), $\delta$ (ppm): 7,11-7,19 (1H, dd, J=6,4/14,0 Hz, $CH_2$=CH); 5,69 (1H, s, N-H); 4,68-4,74 (1H, dd, J=1,3/14,0 Hz, $C\underline{H_2}$=CH, trans); 4,39-4,42 (1H, dd, J=1,2/6.3 Hz, $C\underline{H_2}$=CH, cis); 4,04-4,16 (6H, m, $C\underline{H_2}$-$CH_3$. P-$OC\underline{H_2}$); 3,45-3,51 (2H, m, $C\underline{H_2}$NH); 1,29-1,35 (6H, m, $CH_2$-$C\underline{H_3}$).

Elementaranalyse ($C_9H_{18}NO_6P$:): ber. C: 40,42, H: 6,74, N: 5,24;
gef. C: 40,10, H: 6,61, N: 4,99.

Synthesebeispiel 29: Synthese von Ethyl-bis[2-(vinyloxycarbonylamino)ethyl]-phosphat (EBVCAEP)

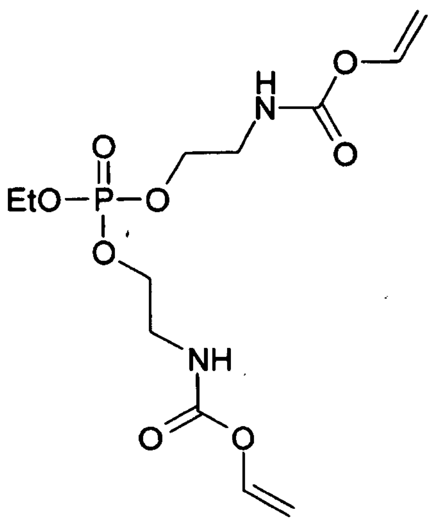

Herstellung analog zu Synthesebeispiel 28 aus 2 Äquivalenten 2-Hydroxyethylaminvinylcarbamat und 1 Äquivalent Dichlorethylphosphinat. Das Rohprodukt wurde säulenchromatographisch (PE:EE = 1:5) auf Kieselgel gereinigt. Es wurden 1,05 g (43 % d. Th.) der Titelverbindung als farbloses, hochviskoses Öl erhalten.
$R_f$ = 0,23 (PE:EE = 1:5)
$^1$H-NMR ($CDCl_3$), δ (ppm): 7,11-7,21 (2H, dd, J=6,3/14,3 Hz, $CH_2$=C<u>H</u>); 5,76 (2H, s, N-H); 4,69-4,76 (2H, dd, J=1,4/14,1 Hz, C<u>$H_2$</u>=CH, trans); 4,40-4,44 (2H, dd, J=1,6/6,3 Hz, C<u>$H_2$</u>=CH, cis); 4,04-4,19 (6H, m, C<u>$H_2$</u>-$CH_3$, POC<u>$H_2$</u>-$CH_2$); 3,44-3,51 (4H, m, C<u>$H_2$</u>NH); 1,26-1,35 (3H, m, $CH_2$-C<u>$H_3$</u>).

Elementaranalyse ($C_{12}H_{21}N_2O_8P$:): ber. C: 40,91, H: 6,01, N: 7,95;
gef. C: 40,63, H: 5,70, N: 7,90.

<u>Synthesebeispiel 30: Synthese von Diethylvinylphosphat (DEVP)</u>

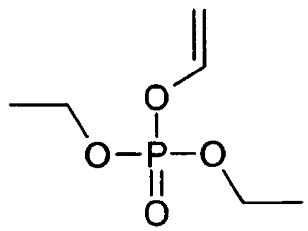

10 ml N-Butyllithium-Lösung (2,1 M Lösung in Hexan) wurden unter Argonatmosphäre bei 0 °C zu 50 ml absolutem THF zugetropft. Die Lösung wurde 30 min lang bei 0°C und 16 h lang bei Raumtemperatur gerührt, anschließend zu 3,01 ml Diethylchlorphosphonat (20 mmol) bei -76°C zugesetzt und 1 h lang bei 0°C, dann 16 h lang bei Raumtemperatur gerührt. Ein weißer Feststoff wurde abfiltriert und das Lösungsmittel am Rotationsverdampfer entfernt. Das leicht gelbliche Rohprodukt wurde säulenchromatographisch (PE:EE = 4:1) auf Kieselgel gereinigt. Es wurden 2,2 g (60 % d. Th.) der Titelverbindung als hellgelbe Flüssigkeit erhalten.
$R_f$ = 0,48 (PE:EE = 4:1)
$^1$H-NMR ($CDCl_3$), δ (ppm): 6,51-6,61 (1H, dd, J=6,3/12,6 Hz, $CH_2$=C<u>H</u>); 4,85-4,91 (1H, dd, J=1,2/12,6 Hz, C<u>$H_2$</u>=CH, trans); 4,53-4,57 (1H, dd, J=1,2/6,3 Hz, C<u>$H_2$</u>=CH, cis); 4,09-4,21 (4H, m, C<u>$H_2$</u>-$CH_3$); 1,29-1,37 (6H, m, $CH_2$-C<u>$H_3$</u>).

<u>Synthesebeispiel 31: Synthese von Divinylethylphosphat (DVEP)</u>

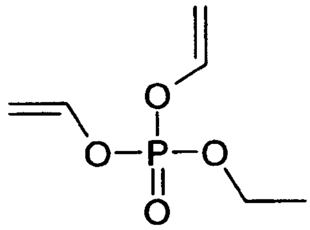

Die Synthese erfolgte analog zu Synthesebeispiel 30 aus n-Butyllithium-Lösung (2,1 M Lösung in Hexan)/THF und Dichlorethylphosphinat. Das dunkelgelbe Rohprodukt wurde säulenchromatographisch (PE:EE = 3:1) auf Kieselgel gereinigt. Es wurden 1,9 g (36 % d. Th.) der Titelverbindung als gelbe Flüssigkeit erhalten.

$R_f$ = 0,42 (PE : EE = 3:1)

$^1$H-NMR ($CDCl_3$), δ (ppm): 6,48-6,65 (2H, dd, J=1,4/6,6 Hz, $CH_2$=C<u>H</u>); 4,87-5,01 (2H, dd, J=1,1/13,5 Hz, C<u>H</u>$_2$=CH, trans); 4,57-4,68 (2H, m, C<u>H</u>$_2$=CH, cis); 4,14-4,32 (2H, m, C<u>H</u>$_2$-$CH_3$); 1,30-1,45 (3H, m, $CH_2$-C<u>H</u>$_3$).

Elementaranalyse ($C_6H_{11}O_4P$): ber. C: 40,46, H: 6,22;
gef. C: 40,68, H: 6,11.

<u>Synthesebeispiel 32: Synthese von Trivinylphosphat (TVP)</u>

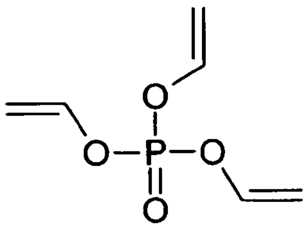

Die Synthese erfolgte analog zu Synthesebeispiel 30 aus n-Butyllithium-Lösung (2,1 M Lösung in Hexan)/THF und Phosphorylchlorid. Das orange-gelbe Rohprodukt wurde säulenchromatographisch (PE:EE = 5:1) auf Kieselgel gereinigt. Es wurden 1,0 g (26 % d. Th.) der Titelverbindung als dunkelgelbe Flüssigkeit erhalten.

$R_f$ = 0,43 (PE : EE = 5:1)

$^1$H-NMR ($CDCl_3$), δ (ppm): 6,46-6,66 (3H, m, $CH_2$=CH); 4,86-5,09 (3H, m, C<u>H</u>$_2$=CH, trans); 4,57-4,77 (3H, m, C<u>H</u>$_2$=CH, cis).

<u>Synthese höhermolekularer Verbindungen</u>

Die Synthese von Verbindungen, in denen $R^1$ ein n-wertiger Rest eines biologisch abbaubaren, bioverträglichen Oligo- oder Polymers ist, z.B. Vinylester von Naturprodukten, erfolgt analog zur Herstellung der entsprechenden Vinylester, Vinylcarbonate und Carbamate.

Beispielsweise können OH-Gruppen enthaltende Polymere, z.B. Polysaccharide wie Glykogen, Amylose, Cellulose oder Hydroxyethylcellulose, in einem geeigneten Lösungsmittel, z.B. DMA/LiCl mit Chlorameisensäurevinylester umgesetzt werden. Analog zur Synthese der Polylactat-Blockcopolymere DEG(PLAVC)$_2$ aus Synthesebeispiel 6 können sämtliche OH-terminierten Polyester (z.B. OH-terminierte Polyglykolsäure) und Polyether (z.B. PEG 2000) zu den entsprechenden Carbonaten umgesetzt werden.

Die freien Aminogruppen an den Lysineinheiten von Gelatine reagieren mit Chlorameisensäurevinylester spontan zu den entsprechenden Carbamaten. Analog kann eine Reihe weiterer Polypeptide und Proteine in Carbamate übergeführt werden. Auch Chitosan kann umgesetzt werden, wobei bei geeigneter Wahl der Äquivalente an Chlorameisensäurevinylester die Amino- selektiv vor den OH-Gruppen reagieren.

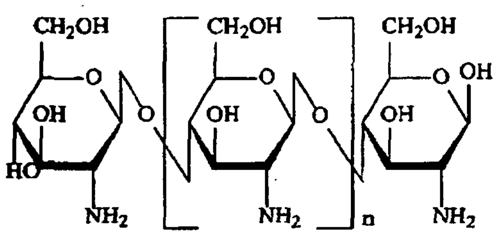

Chitosan

Ebenso kann Chitosan für die Herstellung von Vinylestern genutzt werden. Hierfür werden beispielsweise die freien Aminogruppen mit Vinylacrylat umgesetzt, wobei diese unter Michael-Addition mit der Acrylat-Doppelbindung reagieren. Eine andere Möglichkeit, zu Vinylestern zu gelangen ist die Pd(II)-katalysierte Umsetzung von Carboxyethylcellulose analog zum Erhalt von TUVE aus Trioxaundecandisäure in Synthesebeispiel 7.

Vinylester auf Schwefelbasis können nach einem beliebigen der oben erwähnten Verfahren beispielsweise aus Thiolen erhalten werden, am einfachsten wiederum durch Umsetzung des Thiols, z.B. eines Polypeptids mit Cysteinresten, mit Chlorameisensäurevinylester.

Ebenfalls kann auch die freie P-OH-Gruppe von Phospholipiden wie z.B. Phosphatidylcholinen zuerst mit Oxalylchlorid unter milden Bedingungen zum entsprechenden Säurechlorid umgesetzt werden. Der gewünschte Vinylester des Phospholipids kann nachfolgend durch Umsetzung analog zu Beispiel 30 und Beispiel 31 dargestellt werden.

Somit kann auf einfache Weise eine Vielzahl von Polysacchariden, Polypeptiden, Polyamiden, Polyestern, Polycarbonaten, Polyethern und Fettsäure-Derivaten, die leicht umzusetzende Funktionalitäten wie Hydroxy-, Thiol- und/oder Aminogruppen aufweisen, in die entsprechenden Vinylester und anschließend in eine polymerisierbare Zusammensetzung übergeführt werden, um gewünschte biologisch abbaubare, bioverträgliche, vernetzte Polymere zu ergeben, die sich ihrerseits beispielsweise für einen Einsatz als Knochen- oder Gewebestütz- oder -ersatzmaterial eignen.

Die Löslichkeit des Ausgangspolymers wird durch die Überführung in die entsprechenden Vinylester üblicherweise deutlich verbessert, allerdings werden praktisch durchwegs Feststoffe erhalten. Für einen Einsatz als photopolymerisierbare Formulierung sind daher besonders in diesen Fällen flüssige Comonomere und/oder Lösungsmittel in mehr oder weniger hohen Anteilen erforderlich. Weniger als 5%ige (z.B. 1%ige) Lösungen der Monomere im jeweiligen Lösungsmittel sind zwar ebenfalls möglich, erfindungsgemäß aber nicht bevorzugt, da diese langsame Polymerisationsraten ergeben und zumeist einen hohen Energieaufwand zur anschließenden Entfernung des Lösungsmittels erfordern. Eine Formgebung in Rapid-Prototyping-Verfahren wäre damit ebenfalls nur schwer möglich. Freilich besteht die Möglichkeit, die Viskosität stark verdünnter Monomer-Lösungen mittels Verdickern auf praktikable Werte zu erhöhen, was jedoch ebenfalls nicht bevorzugt ist.

In den nachstehenden Ausführungsbeispielen der Erfindung werden daher bevorzugte Ausführungsformen der Zusammensetzungen beschrieben, die ausschließlich (zäh)flüssige Vinylester-Monomere und Initiatoren enthalten. Die Auswahl der Art und Menge gegebenenfalls zuzusetzender Lösungsmittel bzw. Additive, wie sie zuvor bereits ausführlich erläutert wurden, ist vom durchschnittlichen Fachmann ohne übermäßiges Experimentieren durchführbar.

Beispiele 1 bis 50: Herstellung von Zusammensetzungen

Mono- und difunktionelle Vinylester wurden als Monomere der Formeln (I) bis (III) - in zwei Fällen zusammen mit dem in Synthesebeispiel 5 hergestellten Comonomer 2-Oxo-1,3-dioxolan-4-yl)methylmethacrylat (MC) - zu Zusammensetzungen formuliert, indem sie mit einem der folgenden UV-Photoinitiatoren (A) bis (C) vermischt wurden:

Initiator A: 0,5 Gew.-% Irgacure 819 (Ciba)
Intiator B: 1 Gew.-% Campherchinon und 4-Dimethylaminobenzoesäureethylester (CC/DMAB) im Molverhältnis 1:1
Initiator C: 2 Gew.-% Darocur 1173 (Ciba)

In Beispiel 19 wurde die Oberfläche einer aus AVE-MC-Copolymer bestehenden, gehärteten Probe folgendermaßen mit alkalischer Phosphatase (ALP) als Beispiel für ein Enzym als bioaktiver Wirkstoff modifiziert:

Ein Polymerplättchen mit 1,3 cm Durchmesser wurde in 3 ml ALP-Lösung (2 mg/ml) eingelegt und in 0,05 M Tris-HCl-Puffer bei pH 8 und 4 °C 16 h lang geschüttelt. Das Plättchen wurde mehrmals mit der Pufferlösung gewaschen, und freies, nichtumgesetztes Carbonat MC wurde mit Ethanolamin umgesetzt.

Einen allgemeinen Überblick über Enzymimmobilisierung an Polymeren mit zyklischen Carbonaten an der Oberfläche gibt D.C. Webster, "Cyclic carbonate functional polymers and their applications", Progress in Organic Coatings 47(1), 77-86 (2003).

Auf diese Weise wurden in den nachstehend angeführten Beispielen 1 bis 50 die jeweiligen Zusammensetzungen B1 bis B50 erhalten, wobei die Beispiele 1-3, 28-29, 37, 39-41, 46 und 48, in denen n = 1 ist, nicht im Schutzumfang der vorliegenden Erfindung liegen, sondern lediglich zur Veranschaulichung angeführt sind.

| | | |
|---|---|---|
| Beispiel 1: Decansäurevinylester, DVE | n = 1 | Initiator A |
| Beispiel 2: Hexansäurevinylester, HVE | n = 1 | Initiator A |
| Beispiel 3: N-Acetylphenylalaninvinylester, PAVE | n = 1 | Initiator A |
| Beispiel 4: Adipinsäuredivinylester, AVE | n = 2 | Initiator A |
| Beispiel 5: Sebacinsäuredivinylester, SEVE | n = 2 | Initiator A |
| Beispiel 6: Korksäuredivinylester, KVE | n = 2 | Initiator B |
| Beispiel 7: Adipinsäuredivinylester, AVE | n = 2 | Initiator B |
| Beispiel 8: Sebacinsäuredivinylester, SEVE | n = 2 | Initiator B |
| Beispiel 9: Korksäuredivinylester, KVE | n = 2 | Initiator B |
| Beispiel 10: AVE:HVE = 1:1 | | Initiator B |
| Beispiel 11: AVE: HVE = 3:1 | | Initiator B |
| Beispiel 12: AVE:DVE = 1:1 | | Initiator B |
| Beispiel 13: AVE:DVE = 3:1 | | Initiator B |
| Beispiel 14: AVE:PAVE = 1:1 | | Initiator B |
| Beispiel 15: AVE:PAVE = 3:1 | | Initiator B |
| Beispiel 16: AVE:DVMPL = 1:1 | | Initiator B |
| Beispiel 17: AVE:DVMPL = 3:1 | | Initiator B |
| Beispiel 18: AVE:MC = 20:1 | | Initiator B |
| Beispiel 19: AVE:MC = 20:1 plus Oberflächenmodifizierung | | Initiator B |
| Beispiel 20: Trimerfettsäuretrivinylester, TFVE | n = 3 | Initiator A |
| Beispiel 21: Trioxaundecansäuredivinylester, TUVE | n = 2 | Initiator A |
| Beispiel 22: Ethylenglykol-bis(vinylcarbonat), EGDVC | n = 2 | Initiator A |
| Beispiel 23: Butandiol-bis(vinylcarbonat), BDDVC | n = 2 | Initiator A |
| Beispiel 24: Hexandiol-bis(vinylcarbonat), HDDVC | n = 2 | Initiator A |
| Beispiel 25: Glycerin-tris(vinylcarbonat), GTVC | n = 3 | Initiator A |
| Beispiel 26: Diethylenglykol-bis(vinylcarbonat), DEGDVC | n = 2 | Initiator A |
| Beispiel 27: Polyethylenglykol-bis(vinylcarbonat), PEGDVC | n = 2 | Initiator A |
| Beispiel 28: 2-Cyanoethylvinylcarbonat, CEVC | n = 1 | Initiator A |
| Beispiel 29: Ethylvinylcarbonat, EVC | n = 1 | Initiator A |
| Beispiel 30: Ricinusöl-tris(vinylcarbonat), RiTVC | n = 3 | Initiator A |
| Beispiel 31: hydriertes Ricinusöl-tris(vinylcarbonat), HRiTVC | n = 3 | Initiator A |
| Beispiel 32: Diethylenglykol-bis[O-(O'-vinyloxycarbonyl)polylactat], DEG(PLAVC)$_2$ | n = 2 | Initiator A |
| Beispiel 33: N,N'-Dimethylethylendiamin-bis(vinylcarbamat), DMEDDVCA | n = 2 | Initiator A |
| Beispiel 34: Piperazin-bis(vinylcarbamat), PDVCA | n = 2 | Initiator A |
| Beispiel 35: Ethylendioxy-bis(propylamin)divinylcarbamat, JAVM | n = 2 | Initiator A |
| Beispiel 36: Bis[aminopolyethylenglykol(500)]amin-trivinylcarbamat, EAVM | n = 3 | Initiator A |
| Beispiel 37: Sarkosinmethylestervinylcarbamat, SMEVCA | n = 1 | Initiator A |
| Beispiel 38: N,O-Bis(vinyloxcarbonyl)-N-methylhydroxylamin, MHADVC | n = 2 | Initiator C |
| Beispiel 39: N-Methoxyvinylcarbamat, MVCA | n = 1 | Initiator C |
| Beispiel 40: N-Acryloyl-N-methoxyvinylcarbamat, AMVCA | n = 1 | Initiator C |
| Beispiel 41: Vinyloxycarbonylphosphonsäurediethylester, VCPDE | n = 1 | Initiator C |
| Beispiel 42: EGDVC:CEVC = 5:1 | | Initiator A |
| Beispiel 43: EGDVC:EVC = 5:1 | | Initiator A |
| Beispiel 44: DMEDDVCA:PDVCA = 5:1 | | Initiator A |
| Beispiel 45: DMEDDVCA:SMEVCA = 5:1 | | Initiator A |
| Beispiel 46: 2-(Diethoxyphosphoryloxy)ethylamin-vinylcarbamat, EPEVC | n = 1 | Initiator A |

(fortgesetzt)

| | | |
|---|---|---|
| Beispiel 47: Ethyl-bis[2-(vinyloxycarbonylamino)ethyl]phosphat, EBVCAEP | n = 2 | Initiator A |
| Beispiel 48: Diethylvinylphosphat, DEVP | n = 1 | Initiator A |
| Beispiel 49: Divinylethylphosphat, DVEP | n = 2 | Initiator A |
| Beispiel 50: Trivinylphosphat, TVP | n = 3 | Initiator A |

Vergleichsbeispiele 1 bis 8: Herstellung von Referenzzusammensetzungen

Analog zu den obigen Beispielen wurden anstelle der Vinylester-Monomere andere photopolymerisierbare Monomere mit Initiator vermischt, um die Referenzzusammensetzungen V1 bis V8 zu erhalten, die als Vergleichsbeispiele den Stand der Technik repräsentieren.

| | |
|---|---|
| Vergleichsbeispiel 1: 1,6-Hexandioldiacrylat, HDDA | InitiatorA |
| Vergleichsbeispiel 2: Trimethylolpropantriacrylat, TTA | Initiator A |
| Vergleichsbeispiel 3: ethoxyliertes TTA, MG 1200, ETA | Initiator A |
| Vergleichsbeispiel 4: Polyethylenglykoldiacrylat, MG 800, PEG-DA | Initiator A |
| Vergleichsbeispiel 5: 1,4-Butandioldimethacrylat, BDMA | InitiatorA |
| Vergleichsbeispiel 6: 1,6-Hexandioldiacrylat, HDDA | Initiator B |
| Vergleichsbeispiel 7: Polyethylenglykoldiacrylat, MG 800, PEG-DA | Initiator B |
| Vergleichsbeispiel 8: TTA:ETA = 1:1 | Initiator B |

Härtungstests

Hierfür wurden die wie zuvor beschrieben erhaltenen Zusammensetzungen B1 bis B6, B20 bis B41 und B46 bis B50 sowie die Vergleichszusammensetzungen V1 bis V5 verwendet. Für Phtoto-DSC-Messungen wurden jeweils ca. 5 mg davon in ein DSC-Schälchen aus Aluminium genau eingewogen und das Schälchen auf den rechten Sensor der Messzelle gesetzt, die 5 min lang mit Stickstoff gespült wurde. Ein Schälchen mit einer bereits auspolymerisierten Probe der jeweiligen Zusammensetzung am linken Sensor diente als Bezug. Die Aufzeichnung des DSC-Geräts wurde 2,0 min nach dem Aufsetzen des Schälchens gestartet, und nach Ablauf von 1,0 min wurde mit der Bestrahlung begonnen. Als Strahlungsquelle diente ein Lichtleiter (EXFO Omnicure Series 2000) mit einem UV-Filter im Wellenlängenbereich $\lambda$ = 320-500 nm. Nach Konstanz der DSC-Linie wurde die Messung abgebrochen. Alle Messungen wurden unter Stickstoff durchgeführt.

Als Ergebnis der DSC-Messung wurde der Zeitpunkt des Wärmeflussmaximums $t_{max}$ (entspricht der Zeit bis zum Erreichen der höchsten Polymerisationsrate in [s]), die Fläche des Peaks $\Delta H$ (entspricht der frei gewordenen Polymerisationswärmemenge in [J/g]) und die Höhe des Peaks h (in [mW/mg]) bestimmt. Aus der Fläche des Peaks $\Delta H$, dem Molekulargewicht MG der Monomere und der theoretischen Polymerisationswärme ($\Delta H_0$) der jeweiligen Monomere wurde der Doppelbindungsumsatz (DBC) der einzelnen Messungen nach folgender Gleichung (1) berechnet:

$$DBC \quad [\%] \quad = \frac{\Delta H}{\Delta H_0} \cdot 100 \qquad (1)$$

$\Delta H$ Polymerisationswärme [J/mol] (Fläche des Peaks)

$\Delta H_0$ Theoretische Polymerisationswärme der Einzelkomponente [J/mol]

Weiters kann aus der Peakhöhe, der theoretischen Polymerisationswärme und der Dichte des Harzes $\rho$ die Polymerisationsrate $R_p$ wie folgt berechnet werden (Formel 2):

$$R_P = \frac{h \times \rho}{\Delta H_{0P}} \qquad (2)$$

$R_P$ Polymerisationsrate [mol $l^{-1}$ $s^{-1}$]
h Höhe des Peaks [mW/mg]
ρ Dichte des Harzes [g/dm$^3$]

Die Ergebnisse der Messungen sind in nachstehender Tabelle 1 angegeben.

Tabelle 1 - Ergebnisse für $t_{max}$, Rp und DBC der einzelnen Monomere

| **Beispiel - Monomer** | **Funktio nalitäten** | **$t_{max}$ [s]** | **$R_p$ x $10^3$ [mol/l.s]** | **DBC [%]** |
|---|---|---|---|---|
| B1 - DVE | 1 | 35 | 36 | 58 |
| B2 - HVE | 1 | 37 | 35 | 44 |
| B3 - PAVE | 1 | 32 | 48 | 52 |
| B4 - AVE | 2 | 15 | 198 | 79 |
| B5 - SEVE | 2 | 22 | 99 | 82 |
| B6 - KVE | 2 | 15 | 173 | 86 |
| B20 - TFVE | 3 | 33 | 23 | 52 |
| B21 - TUVE | 2 | 37 | 105 | 85 |
| B22 - EGDVC | 2 | 12 | 213 | 79 |
| B23 - BDDVC | 2 | 14 | 173 | 80 |
| B24 - HDDVC | 2 | 17 | 150 | 83 |
| B25 - GTVC | 3 | 9 | 75 | 45 |
| B26 - DEGDVC | 2 | 17 | 127 | 68 |
| B27 - PEGDVC | 2 | 28 | 43 | 62 |
| B28 - CEVC | 1 | 21 | 59 | 75 |
| B29 - EVC | 1 | 23 | 47 | 68 |
| B30 - RiTVC | 3 | 16 | 67 | 51 |
| B31 - HRiTVC | 3 | 14 | 82 | 89 |
| B32 - DEG(PLAVC)$_2$ | 2 | 14 | 65 | 52 |
| B33 - DMEDDVCA | 2 | 16 | 117 | 74 |
| B35 - JAVM | 2 | 25 | 186 | 74 |
| B36 - EAVM | 3 | 36 | 23 | 98 |
| B37 - SMEVCA | 1 | 21 | 75 | 76 |
| B38 - MHADVC | 2 | 10 | 91 | 70 |
| B39 - MVCA | 1 | 14 | 73 | 81 |
| B40 - AMVCA | 1(2) | 6 | 165 | 82 |
| B41 - VCPDE | 1 | 11 | 155 | 95 |
| B46 - EPEVC | 1 | 14 | 61 | 90 |
| B47 - EBVCAEP | 2 | 7 | 74 | 78 |
| B48 - DEVP | 1 | 20 | 33 | 90 |
| B49 - DVEP | 2 | 27 | 127 | 89 |
| B50 - TVP | 3 | 23 | 10 | 75 |
| V1 - HDDA | 2 | 7 | 247 | 87 |
| V2 - TTA | 3 | 5 | 98 | 47 |

(fortgesetzt)

| Beispiel - Monomer | Funktio nalitäten | $t_{max}$ [s] | $R_p$ x $10^3$ [mol/l.s] | DBC [%] |
|---|---|---|---|---|
| V3 - ETA | 3 | 6 | 46 | 78 |
| V4 - PEG-DA | 2 | 5 | 98 | 94 |
| V5 - BDMA | 2 | 22 | 91 | 51 |

Man erkennt, dass die Zusammensetzungen der Beispiele, in denen difunktionelle Monomere der Formel (I) zum Einsatz kamen, im Allgemeinen mit guten bis sehr guten Polymerisationsraten Rp härten. Die meisten monofunktionellen Monomere härteten erwartungsgemäß langsamer, liegen allerdings dennoch im Bereich der di- und trifunktionellen Vergleichsbeispiele. Deutlich langsamer härten lediglich die Monomere der Beispiele 20, 36 und vor allem 50, was im Falle der beiden Ersteren auf die geringe Beweglichkeit der hier verwendeten hochmolekularen Monomere und die dadurch ebenfalls gegebene große Entfernung zwischen den Vinylestergruppen und im Falle von Beispiel 50 - obwohl trifunktionell - auf eine stabilisierende Wirkung des Triphosphats zurückzuführen sein dürfte. Etwas überraschend ist angesichts dessen die sehr gute Leistungsfähigkeit des difunktionellen Phosphatesters aus Beispiel 49, aber auch der "monofunktionellen" Monomere der Beispiele 40 und 41. Dies dürfte für die beiden Letzteren einerseits der Gegenwart einer zusätzlichen Acryloylgruppe in Beispiel 40 (weshalb das Monomer in diesem Versuch eigentlich difunktionell ist) und andererseits der Phosphonsäure-Gruppierung bzw. dem geringen Molekulargewicht in Beispiel 41 zuzuschreiben sein. Vergleicht man Monomere mit gleicher Größe und Anzahl an funktionellen Gruppen wie etwa B22 und V5 bzw. B23 und V1, so ist leicht ersichtlich, dass die Reaktivität der neuen Monomere zwischen jener der hochreaktiven Acrylate und jener der bisher für Implantate üblicherweise eingesetzten Methacrylate liegt. Nahezu alle Zusammensetzungen der erfindungsgemäßen Beispiele erreichen jedoch die Polymerisationsraten der Vergleichsbeispiele und übertreffen diese in vielen Fällen sogar.

Die Werte für $t_{max}$ sind bei den Zusammensetzungen der erfindungsgemäßen Beispiele zwar großteils höher als jene der meisten Vergleichsbeispiele (mit Ausnahme von V5, einem Methacrylat wobei diese funktionelle Gruppe in der Praxis den Acrylaten zumeist vorgezogen wird), liegen aber in einem für die praktische Umsetzung der Erfindung durchaus annehmbaren Bereich, zumal in bevorzugten Zusammensetzungen ohnehin zumindest 35 %, noch bevorzugter zumindest 50 %, di- oder polyfunktionelle und damit rasch härtende Vinylester als Vernetzer eingesetzt werden. Die Beispiele 40, 47 und 25 zeigen hier die beste Leistungsfähigkeit der Zusammensetzungen und liegen im Bereich der am schnellsten startenden Gemische der Vergleichsbeispiele.

Die Doppelbindungsumsätze DBC aller getesteten Zusammensetzungen liegen im Bereich jener der Vergleichsbeispiele, wobei das Phosphonsäure-Derivat aus Beispiel 41 den besten Wert liefert. Auffällig ist weiters, dass die beiden difunktionellen Vinylphosphate der Beispiele 48 und 49 ebenfalls sehr hohe DBC-Werte liefern, aber auch der trifunktionelle Phosphatester aus Beispiel 50 schneidet überdurchschnittlich gut ab. Die getesteten Zusammensetzungen sind somit zur Herstellung kommerzieller Produkte auf wirtschaftliche Weise durchaus geeignet. Darüber hinaus wurde gezeigt, dass als vergleichsweise wenig reaktiv bekannte Vinylester - auch als Carbonate, Carbamate und Phosphate - bei Masse-Photopolymerisation überraschend hohe Reaktivität, ähnlich jener der als Messlatte dienenden und kommerziell weit verbreiteten (Meth)acrylate besitzen.

Toxizitätstests

*A) Osteoblastenzellen*

Für die Prüfung auf Toxizität wurden osteoblastenähnliche Zellen MC3T3-E1 (Quelle ATCC CRL-2596) verwendet. Die adhärenten Zellen wurden zunächst mit Pronase voneinander und vom Boden der Petrischalen gelöst und anschließend mit frisch bereitetem Nährmedium, bestehend aus im Handel erhältlichem Minimal Essential Medium Eagle's alpha Modification (αMEM), das mit zusätzlicher Glucose von ursprünglich 1 g/l auf eine Glucose-Konzentration von 4,5 g/l sowie mit 10 % FKS (fötales Kälberserum), 30 μg/ml Gentamycin (Breitbandantibiotikum), L-Glutamin (400 mg/l) und Ascorbinsäure (50 mg/l) ergänzt worden war, auf eine Zellkonzentration von 40.000 Zellen/ml vermischt. Jeweils 1 ml dieser Zellsuspension wurde in den Wells (DM 1,9 cm) von Multiwell-Platten vorgelegt.

Das Multiwell mit den Zellen wurde 5 d lang bei 37 °C in feuchter Atmosphäre mit 5 % $CO_2$ mit zunehmenden Mengen der in den Beispielen und Vergleichsbeispielen eingesetzten Monomere inkubiert. Anschließend wurden die Zellen mit phosphatgepufferter physiologischer Kochsalzlösung gewaschen und vor der Messung zum Aufbrechen der Zellen eingefroren. Nach dem Auftauen wurde die Menge an Desoxyribonukleinsäure, die proportional zur Zellanzahl ist, durch Färbung mit einem Fluoreszenzfarbstoff, Messen der Fluoreszenz bei 460 nm (nach Anregung bei 360 nm) und Vergleich mit einer zuvor erstellten Eichkurve bestimmt. Die interpolierte Konzentration, bei der im Vergleich zur

Kontrolle die Hälfte der Zellen überlebt hatten, wurde als "In-vitro-LC50" bezeichnet. Die Ergebnisse sind in folgender Tabelle 2 angeführt.

Tabelle 2 - Ergebnisse der Toxizitätstests mit Osteoblasten

| **Monomer** | **In-vitro-LC50 x $10^4$ [mol/l]** |
|---|---|
| DVE | > 100 |
| HVE | > 100 |
| PAVE | > 100 |
| AVE | > 100 |
| SEVE | > 100 |
| KVE | > 100 |
| DVMPL | > 100 |
| TFVE | > 100 |
| TUVE | > 100 |
| EGDVC | > 100 |
| BDDVC | > 100 |
| HDDVC | > 100 |
| GTVC | > 100 |
| DEGDVC | > 100 |
| PEGDVC | > 100 |
| CEVC | > 100 |
| EVC | > 100 |
| HRiTVC | > 100 |
| $DEG(PLAVC)_2$ | > 100 |
| DMEDDVCA | > 100 |
| JAVM | > 100 |
| EAVM | > 100 |
| SMEVCA | > 100 |
| MHADVC | > 100 |
| MVCA | > 100 |
| AMVCA | < 0,1 |
| VCPDE | > 100 |
| EPEVC | > 100 |
| EBVCAEP | > 100 |
| DEVP | > 100 |
| DVEP | > 100 |
| TVP | > 100 |
| Verbgleich: HDDA | < 0,1 |
| Vergleich: TTA | < 0,1 |
| Vergleich: ETA | 0,7 |
| Vergleich: PEG-DA | 1,1 |

(fortgesetzt)

| Monomer | In-vitro-LC50 x $10^4$ [mol/l] |
|---|---|
| Vergleich: BDMA | < 0,1 |

Die Tabelle zeigt, dass die in den Zusammensetzungen eingesetzten Vinylester-Monomere um zumindest zwei Größenordnungen weniger toxisch für Osteoblasten sind als die Acrylate der Vergleichsbeispiele. Die einzige Ausnahme stellt das Monomer AMVCA, N-Acryloyl-N-methoxyvinylcarbamat, dar, das aufgrund der darin enthaltenen Acryloylgruppe für die Osteoblasten ähnlich toxisch ist wie die Mehrzahl der Vergleichsbeispiele. Dies unterstreicht und bestätigt die Zielsetzung der Erfindung, toxische Acrylate als Monomere für in vivo zu verwendende Polymere zu vermeiden. Die neue Verbindung N-Acryloyl-N-methoxyvinylcarbamat, die aufgrund ihrer Polymerisationseigenschaften ein wertvolles Monomer für vielerlei Anwendungen ist, ist daher in den in Anspruch 1 definierten Zusammensetzungen insofern nur bedingt einsetzbar, als dafür Sorge getragen werden muss, dass keinerlei Restmonomere im fertigen Polymerprodukt enthalten sind. Dies kann beispielsweise mittels einer Nachbehandlung, z.B. Extraktion, Umfällung oder dergleichen, des Polymers erfolgen.

*B) Endothelzellen*

Zur zusätzlichen Toxizitätstestung von Monomeren der Formel (I) wurden humane venöse Nabelschnur-Endothelzellen (HUVEC) verwendet. Nach Trypsinisierung der konfluenten Primärkulturen wurden die Zellen in handelsüblichem Medium 199 mit 20% fötalem Kälberserum suspendiert, in 96-Well-Zellkulturplatten mit einer Konzentration von 40.000 Zellen/$cm^2$ ausgesiedelt und erneut bis zur Konfluenz kultiviert (37 °C, 5 % $CO_2$). Anschließend wurden die Zellüberstände abgehoben und die Endothelzellen mit steigenden Konzentrationen der Monomere (0,1 nM bis 1 mM in Medium 199 mit 10 % fötalem Kälberserum) für 24 h kultiviert. Die Beeinflussung der Zellproliferation wurde mittels eines nichtradioaktiven Zellproliferations- und Zytotoxizitätstest (EZ4U, Fa. Biomedica, Wien) untersucht. Dieser Test beruht auf der Umwandlung farbloser Tetrazoliumsalze in stark gefärbte Formazanderivate durch lebende Zellen. Die photometrisch gemessene Färbung ist proportional zur Anzahl an lebenden Zellen in der Probe. Somit kann der Einfluss der Testsubstanzen auf die Proliferation der Zellen durch Photometrie bestimmt werden. Als Wachstumskontrolle dienten Endothelzellen, die ohne Zusatz von Monomerlösungen kultiviert wurden. In nachstehender Tabelle 3 sind jene Monomerkonzentrationen angegeben, die zu einer halbmaximalen Proliferationshemmung führten $C_{max1/2}$.

Tabelle 3 - Toxizitätstests mit Endothelzellen

| Monomer | $C_{max1/2}$ [μmol/l] |
|---|---|
| DVE | > 1000 |
| HVE | > 1000 |
| PAVE | > 1000 |
| AVE | > 1000 |
| SEVE | > 1000 |
| KVE | > 1000 |
| DVMPL | > 1000 |
| Vergleich: HDDA | 20 |
| Vergleich: TTA | 10 |
| Vergleich: ETA | 100 |
| Vergleich: PEG-DA | 500 |
| Vergleich: BDMA | 40 |

Auch dieser Test belegt eindeutig, dass die in den Zusammensetzungen verwendeten Vinylester-Monomere der Formel (I) von Zellen um zumindest eine Größenordnung besser vertragen werden als Acrylate.

Biokompatibilitätstests

*A) Herstellung der Probekörper*

Zur Überprüfung der Biokompatibilität wurden Probekörper aus den Zusammensetzungen der Beispiele B7 bis B19 und der Vergleichsbeispiele V6 bis V8 hergestellt.

Die Gemische wurden in eine Silikonform zur Herstellung kreisrunder Plättchen gegossen und unter Stickstoffatmosphäre auf einer UV-Anlage (Hg-Hochdrucklampe ungefiltert, 1000 W) ausgehärtet. Die erhaltenen Probekörper wurden zur Entfernung von Restmonomeren mit organischen Lösungsmitteln und Wasser im Ultraschallbad extrahiert. Die extrahierten Polymerkörper wurden durch Bestrahlung mit UV-Licht sterilisiert.

Als weiterer Vergleich, d.h. Vergleichsbeispiel 9, wurde ein im Handel von Sigma Aldrich erhältliches Polycaprolacton mit MG 14000 aufgeschmolzen und in der Silikonform ebenfalls zu einem Plättchen gegossen.

*B) Tests mit Osteoblastenzellen*

Für die Prüfung auf Biokompatibilität wurden osteoblastenähnliche Zellen MG-63 (ATCC CRL-1427) verwendet, die wie zuvor für die Toxizitätstests beschrieben präpariert, in demselben Nährmedium suspendiert und in oben beschriebener Weise auf die Wells von Multiwell-Platten verteilt wurden, in die vorher die Probekörper eingelegt worden waren.

Das Multiwell mit den Zellen wurde 3 d lang bei 37 °C in feuchter Atmosphäre mit 5 % $CO_2$ in Gegenwart der Probekörper inkubiert. Anschließend wurde die metabolische Aktivität der lebenden Zellen (Zellvitalität) mittels des EZ4U-Tests (Fa. Biomedica, Wien) wie zuvor für die Toxizitätstests beschrieben photometrisch bestimmt und mit der Zellaktivität auf Zellkulturschalen in Beziehung gesetzt, wobei als Wachstumskontrolle Zellen dienten, die auf handelsüblichen Kulturschalen kultiviert wurden. In nachstehender Tabelle 4 ist für die jeweiligen Probekörper-Beispiele die entsprechende Anzahl an in der Probe enthaltenen Zellen in Prozent der Kontrolle angegeben (Kontrolle = 100 % Zellen).

Tabelle 4 - Biokompatibilität mit Osteoblasten

| Beispiel - Monomere der Probekörper | Zellanzahl (% der Kontrolle) |
|---|---|
| B7 - AVE | 109 |
| B8 - SEVE | 192 |
| B9 - KVE | 130 |
| B10 - AVE:HVE(1:1) | 92 |
| B11 - AVE:HVE (3:1) | 102 |
| B12 - AVE:DVE(1:1) | 125 |
| B13 - AVE:DVE (3:1) | 130 |
| B14 - AVE:PAVE(1:1) | 128 |
| B15 - AVE:PAVE (3:1) | 112 |
| B16 - AVE:VMDPL (1:1) | 144 |
| B17 - AVE:VMDPL (3:1) | 102 |
| B18 - AVE:MC (20:1) | 124 |
| B19 - AVE:MC (20:1) mod. | 246 |
| V6 - HDDA | 48 |
| V7 - PEG-DA | 24 |
| V8 - TTA:ETA (1:1) | ~ 1 |
| V9 - PCL | ~ 1 |

Aus der Tabelle geht klar hervor, dass die Probekörper der Vergleichsbeispiele eine deutliche Reduktion der Zellanzahl zur Folge hatten, während aus den Zusammensetzungen der erfindungsgemäßen Beispiele hergestellte Probekörper sogar eine Vermehrung der Zellen begünstigten.

*C) Tests mit Endothelzellen*

Für die Biokompatibilitätstests wurden erneut humane venöse Nabelschnur-Endothelzellen (HUVEC) verwendet. Nach Trypsinisierung der konfluenten Primärkulturen wurden die Zellen in Medium 199 mit 20 % fötalem Kälberserum (FCS) suspendiert und auf die zu testenden Probekörper ausgesiedelt (40.000 Zellen/$cm^2$). Nach 24-stündiger Kultivierung der Zellen (37 °C, 5 % $CO_2$ wurden die Zellüberstände abgehoben, die Endothelzellen mit phosphatgepufferter Salzlösung (PBS) gewaschen und mit Medium 199 mit 10 % FCS 1 h lang äquilibriert. Die Zellproliferation wurde anschließend mittels des EZ4U-Tests bestimmt. Als Vergleich wurde die Proliferation der Endothelzellen auf zur Verbesserung der Zellanhaftung speziell vorbehandelten Kunststoff-Deckplättchen ("Cell culture-treated plastic coverslips", Thermanox®, Fa. Nunc) gemessen und als 100 % angenommen. Die Daten sind als Mittelwerte von Mehrfachbestimmungen in Tabelle 5 angegeben.

Tabelle 5 - Biokompatibilität mit Endothelzellen

| Beispiel - Monomere der Probekörper | Zellanzahl (% der Kontrolle) |
|---|---|
| B7 - AVE | 73 |
| B8 - SEVE | 70 |
| B9 - KVE | 69 |
| B10 - AVE:HVE (1:1) | 75 |
| B11 - AVE:HVE (3:1) | 68 |
| B12 - AVE:DVE (1:1) | 63 |
| B13 - AVE:DVE (3:1) | 62 |
| B14 - AVE:PAVE (1:1) | 90 |
| B15 - AVE:PAVE (3:1) | 68 |
| B16 - AVE:VMDPL (1:1) | 110 |
| B17 - AVE:VMDPL (3:1) | 82 |
| B18 - AVE:MC (20:1) | 68 |
| B19 - AVE:MC (20:1) mod. | 194 |
| V6 - HDDA | 59 |
| V7 - PEG-DA | 14 |
| V8 - TTA:ETA (1:1) | 24 |
| V9 - PCL | 1 |

Es zeigt sich, dass auf den aus Zusammensetzungen der erfindungsgemäßen Beispiele hergestellten Polymer-Probekörpern bis zum Doppelten der Zellanzahl eines Kunststoffs angesammelt hatten, der mit seiner vorbehandelten Oberfläche speziell die Anhaftung von Zellen fördert. Mit einer entsprechenden Oberflächenbehandlung wären die Polymere problemlos in der Lage, den Vergleichswert zu übertreffen, was sich in Beispiel 19 zeigt. Im Gegensatz dazu erreichen die aus den Vergleichsbeispielen erzeugten Polymere durchwegs (weitaus) schlechtere Werte. Einzig HDDA kann mit den niedrigsten Werten der Beispiele knapp mithalten.

Die Morphologie adhärenter Endothelzellen auf Probekörpern aus den Zusammensetzungen von Beispiel 7 und Vergleichsbeispiel 2 wurde mittels Rasterelektronenmikroskopie untersucht. Die Aufnahmen mit einer Vergrößerung von 350fach (Fig. 1 a, 1b) bzw. 150fach (Fig. 1 c) sind in Fig. 1 dargestellt. Beim Acrylat-Vergleich in Fig. 1a (Polymer aus Vergleichsbeispiel 2) sind nur vereinzelte Zellen zu erkennen, die sich darüber hinaus kaum am Probekörper angelagert hatten, was an ihrer runden Form zu erkennen ist. Im Gegensatz dazu haften an dem aus der Zusammensetzung B7 hergestellten Kunststoff die Zellen gut an, wie anhand ihrer unregelmäßigen Form in Fig. 1b ersichtlich ist, und es hatten sich zahlreiche Zellen gebunden, was aus Fig. 1c besonders gut hervorgeht.

Mechanische Eigenschaften

Aus den Zusammensetzungen der erfindungsgemäßen Beispiele 7 bis 17, 20 bis 27, 30 bis 32, 33, 35, 36 und

38 bis 45 sowie jenen der Vergleichsbeispiele V1 bis V4, V8 und V9 wurden kreisrunde Probekörper mit 5 mm Durchmesser und 1 mm Höhe geformt, deren mechanische Eigenschaften mittels Nanoindentation auf folgende Weise gemessen wurden.

Die Indentationshärte $H_{IT}$ und der Indentationsmodul $E_{IT}$ wurden mit dem Nanoindenter XP, MTS Systems Inc. bestimmt. Die Probekörper wurden dafür mit einem ZweiKomponentenkleber auf einen Aluminiumblock aufgeklebt und mit Schleifpapieren verschiedener Körnung geschliffen und poliert. Mit einer Diamant-Pyramide nach Berkovich erfolgte der Eindringversuch mit einer Eindringtiefe von 2 μm und einer Eindringgeschwindigkeit von 0,1 μm/s. Nach einer Haltezeit von 30 s bei Maximallast wurden die Probekörper wieder entlastet. Aus der Steigung der Tangente der Entlastungskurve bei Maximallast kann nun der Indentationsmodul $E_{IT}$ berechnet werden:

$$E_{IT} = \frac{1-(\nu_s)^2}{\frac{1}{E_r} - \frac{1-(\nu_i)^2}{E_i}} \quad (3)$$

$v_{s,i}$ Poissonverhältnis der Probe und des Indenters (für alle Proben $v_s$ = 0,35)
$E_i$ Modul des Indenters [MPa]
$E_r$ reduzierter Modul des Indentationskontakts [MPa]
wobei gilt:

$$E_r = \frac{\sqrt{\pi} \cdot S}{2\sqrt{A_p}} \quad (4)$$

S Kontaktfestigkeit [N/m]
$A_p$ projizierte Kontaktfläche [$m^2$]

Die Indentationshärte $H_{IT}$ wurde anhand der Maximalkraft $F_{max}$ berechnet (W.C. Oliver, G.M. Pharr, J. Mater. Res. 7, 1564 (1992), und ISO 14577):

$$H_{IT} = \frac{F_{\max}}{24.5 \cdot h_c^2} \quad (5)$$

$F_{max}$ Maximalkraft [N]
wobei gilt:

$$h_c = h_{\max} - \varepsilon(h_{\max} - h_r) \quad (6)$$

$h_{max}$ Eindringtiefe bei $F_{max}$ [m]
$h_r$ Schnittpunkt der Tangente der Entlastungskurve bei Maximallast mit der x-Achse [m]
ε Indenterkonstante

Die Ergebnisse sind in nachstehender Tabelle 3 als Mittelwerte von Mehrfachbestimmungen angeführt.

Tabelle 3 - Mechanische Eigenschaften

| Beispiel - Monomere der Probekörper | Härte [MPa] | E-Modul [MPa] |
|---|---|---|
| B7 - AVE | 188 | 1937 |
| B8 - SEVE | 113 | 1304 |
| B9 - KVE | 139 | 1496 |

(fortgesetzt)

| Beispiel - Monomere der Probekörper | Härte [MPa] | E-Modul [MPa] |
|---|---|---|
| B10 - AVE:HVE (1:1) | 90 | 1456 |
| B11 - AVE:HVE (3:1) | 126 | 1705 |
| B12 - AVE:DVE (1:1) | 44 | 587 |
| B13 - AVE:DVE (3:1) | 107 | 1287 |
| B14 - AVE:PAVE (1:1) | 128 | 1558 |
| B15 - AVE:PAVE (3:1) | 156 | 1628 |
| B16 - AVE:DVMPL (1:1) | 147 | 1412 |
| B17 - AVE:DVMPL (3:1) | 158 | 1592 |
| B20 - TFVE | 2,5 | 18 |
| B21 - TUVE | 109 | 2335 |
| B22 - EGDVC | 298 | 3910 |
| B23 - BDDVC | 214 | 2553 |
| B24 - HDDVC | 175 | 2017 |
| B25 - GTVC | 396 | 4688 |
| B26 - DEGDVC | 107 | 1605 |
| B27 - PEGDVC | 9 | 187 |
| B30 - RiTVC | 98 | 512 |
| B31 - HRiTVC | 5 | 31 |
| B32 - $DEG(PLAVC)_2$ | 270 | 2992 |
| B33 - DMEDDVCA | 297 | 4344 |
| B35 - JAVM | 142 | 2922 |
| B36 - EAVM | 3,2 | 20 |
| B38 - MHADVC | 319 | 4115 |
| B39 - MVCA | 108 | 1315 |
| B40 - AMVCA | 301 | 3875 |
| B41 - VCPDE | 125 | 1424 |
| B42 - EGDVC:CEVC (5:1) | 152 | 2310 |
| B43 - EGDVC:EVC (5:1) | 142 | 2108 |
| B44 - DMEDDVCA:PDVCA (5:1) | 161 | 2468 |
| B45 - DMEDDVCA:SMEVCA (5:1) | 189 | 2576 |
| B46 - EPEVC | 47 | 446 |
| B47 - EBVCAEP | 167 | 1832 |
| B48 - DEVP | 76 | 669 |
| B49-DVEP | 189 | 2532 |
| B50-TVP | 267 | 2856 |
| V1 - HDDA | 131 | 1791 |
| V2 - TTA | 296 | 3386 |
| V3 - ETA | 17 | 349 |

(fortgesetzt)

| Beispiel - Monomere der Probekörper | Härte [MPa] | E-Modul [MPa] |
|---|---|---|
| V4 - PEG-DA | 11 | 212 |
| V8-TTA:ETA(1:1) | 78 | 691 |
| V9 - PCL | 49 | 722 |

Aus der Tabelle ist zu entnehmen, dass aus den Zusammensetzungen der erfindungsgemäßen Beispiele Polymerkörper mit einer großen Bandbreite unterschiedlicher Härte und Elastizität erzeugt werden können. Durch Zusatz von Comonomeren oder optionalen Additiven, wie z.B. von Weichmachern, Füllstoffen usw., und/oder durch geeignete Nachbehandlung, wie z.B. Wärmebehandlungs- und/oder Extraktionsschritte nach der Polymerisation der Zusammensetzungen, kann diese Vielfalt noch deutlich vergrößert werden. Es ist somit problemlos möglich, die mit den Vergleichsbeispielen erzielbaren Eigenschaften in allen Belangen zu übertreffen, so dass Zusammensetzungen der Beispiele für die vorliegende Erfindung für diverse Einsatzgebiete im oder am menschlichen oder tierischen Körper oder als Beschichtungsmaterialien, z.B. für Medizinprodukte, oder im Lebens- oder Arzneimittelmittelkontakt infrage kommen. Die gewerbliche Anwendbarkeit der Monomere und Zusammensetzungen, z.B. zur Herstellung von Gewebestütz- oder -ersatzmaterial, steht daher außer Zweifel.

## Patentansprüche

**1.** Verwendung einer durch Polymerisation härtbaren Zusammensetzung zur Herstellung biologisch abbaubarer, bioverträglicher, vernetzter Polymere, vorzugsweise solcher auf Basis von Polyvinylalkohol, für Körperimplantate sowie als Gewebe-Stütz- und -Regenerations-Material, wobei die Zusammensetzung Folgendes umfasst:

a) 5 bis 100 Gew.-% eines oder mehrerer Vinylester-Monomere, die unabhängig voneinander aus Verbindungen der allgemeinen Formeln (I) bis (III) ausgewählt werden:

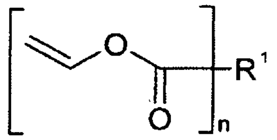

(I)

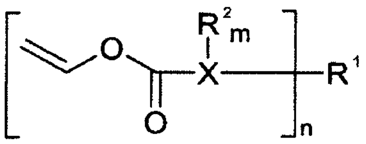

(II)

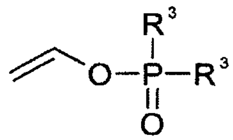

(III)

worin
X ein aus Sauerstoff, Schwefel, Stickstoff und Phosphor ausgewähltes Heteroatom ist;
die n jeweils unabhängig voneinander 1 bis 1000, vorzugsweise 1 bis 50, noch bevorzugter 1 bis 20, noch bevorzugter 1 bis 10, insbesondere 1 bis 3, sind, wobei zumindest 20 % der $n \geq 2$ sind;
die $R^1$ jeweils unabhängig voneinander ausgewählt werden aus

i) Wasserstoff, unverzweigten, verzweigten oder zyklischen, gesättigten oder ungesättigten, n-wertigen Kohlenwasserstoffresten mit 1 bis 30, vorzugsweise 3 bis 25, noch bevorzugter 4 bis 20, insbesondere 5 bis 15, Kohlenstoffatomen, die gegebenenfalls ein oder mehrere, aus Sauerstoff, Schwefel, Stickstoff und Phosphor ausgewählte Heteroatome innerhalb der Kette und/oder am Kettenende aufweisen und gegebenenfalls mit einem oder mehreren Substituenten, ausgewählt aus -OH, -COOH, -CN, -CHO und =O substituiert sind, und
ii) n-wertigen Resten biologisch abbaubarer, bioverträglicher Oligo- und Polymere, ausgewählt aus Polysacchariden, Polypeptiden, Polyamiden, Polyestern, Polycarbonaten, Polyethern und Fettsäurederivaten;

m eine ganze Zahl von 0 bis 4 ist;
die Reste $R^2$ aus Wasserstoff, -OH, =O und den Optionen für $R^1$ ausgewählt werden; und
die Reste $R^3$ aus Wasserstoff, -OH und den Optionen für $R^1$ ausgewählt werden;

b) 0 bis 50 Gew.-% eines oder mehrerer ethylenisch ungesättigter Comonomere, ausgewählt aus (Meth)acrylsäure-, Maleinsäure-, Fumarsäure-, Vinylpyrrolidon- und $\alpha$-Olefin-Monomeren;

c) 0 bis 10 Gew.-% eines oder mehrerer Polymerisationsinitiatoren, ausgewählt aus thermischen Initiatoren und Photoinitiatoren; und

d) 0 bis 95 Gew.-% eines oder mehrerer Lösungsmittel, ausgewählt aus Wasser, niederen Alkoholen, Ether-, Keton-, Ester-, Amid- und Kohlenwasserstoff-Lösungsmit-teln;

wobei die biologisch abbaubaren, bioverträglichen, vernetzten Polymere hergestellt werden, indem die oben definierte Zusammensetzung in eine gewünschte Form gebracht und thermisch oder photochemisch polymerisiert wird.

**2.** Verwendung nach Anspruch 1, **dadurch gekennzeichnet, dass** das zumindest eine Vinylester-Monomer der allgemeinen Formel (I), (II) oder (III) zumindest 50 Mol-% aller enthaltenen Monomere ausmacht.

**3.** Verwendung nach Anspruch 1 oder 2, **dadurch gekennzeichnet, dass** zumindest 35, vorzugsweise zumindest 50, Mol-% aller Vinylester-Monomere di- oder höherfunktionelle, als Vernetzer wirkende Monomere mit $n \geq 2$ sind.

**4.** Verwendung nach einem der Ansprüche 1 bis 3, **dadurch gekennzeichnet, dass** die Zusammensetzung zumindest ein Vinylester-Monomer der Formel (III) umfasst, worin zumindest einer der Reste $R^3$ Vinyloxy ist.

**5.** Verwendung nach einem der vorangegangenen Ansprüche, **dadurch gekennzeichnet, dass** zumindest ein Vinylester-Monomer aus 1,4-Butandiol-bis(vinylcarbonat), 2-Cyanoethylvinylcarbonat, N,N'-Dimethyl-1,2-ethylendiamin-bis(vinylcarbamat), Sarkosinmethylestervinylcarbamat, N,O-Bis(vinyloxycarbonyl)-N-methylhydroxylamin, N-Methoxyvinylcarbamat, N-Acryloyl-N-methoxyvinylcarbamat, Vinyloxycarbonylphosphonsäurediethylester, 2-(Diethoxyphosphoryloxy)ethylamin-vinylcarbamat, Ethyl-bis[2-(vinyloxycarbonylamino)ethyl]phosphat und Divinylethylphosphat ausgewählt wird.

**6.** Verwendung nach einem der vorangegangenen Ansprüche, **dadurch gekennzeichnet, dass** die Comonomere als Komponente b) ausgewählt werden aus: (Meth)-acrylsäureanhydrid, (Meth)acrylsäureglycidylester, (Meth)acryloyloxybemsteinsäure-anhydrid, (Meth)acryloyloxymethylbemsteinsäureanhydrid, (Meth)acrylsäure-2-oxo-1,3-dioxolanylmethylester, Vinylbernsteinsäureanhydrid, Vinylencarbonat und Maleinsäureanhydrid.

**7.** Verwendung nach einem der vorangegangenen Ansprüche, **dadurch gekennzeichnet, dass** die Zusammensetzung als weitere Komponente e) ein oder mehrere Additive, ausgewählt aus Polymerisations-Sensibilisatoren und -Inhibitoren, Stabilisatoren, Modifikatoren, Weichmachern, Färbemitteln, bioaktiven Wirkstoffen, Zellen, wie z.B. Osteoblasten, Verdickem und Füllstoffen, enthält.

**8.** Verwendung nach Anspruch 7, **dadurch gekennzeichnet, dass** die bioaktiven Wirkstoffe aus Arzneimitteln, Proteinen, Antikörpern und Liganden von Zelloberflächenrezeptoren ausgewählt werden.

**9.** Verwendung nach einem der vorangegangenen Ansprüche, **dadurch gekennzeichnet, dass** ein oder mehrere Additive kovalent an Monomere oder Comonomere gebunden werden.

**10.** Verwendung nach Anspruch 9, **dadurch gekennzeichnet, dass** zumindest ein kovalent an Monomere oder Comonomere gebundenes Additiv ein bioaktiver Wirkstoff ist.

**11.** Verwendung nach einem der vorangegangenen Ansprüche, **dadurch gekennzeichnet, dass** ein biologisch abbaubares, bioverträgliches, vernetztes Polymer auf Basis von Polyvinylalkohol durch thermische oder Photopolymerisation der Zusammensetzung hergestellt wird.

**12.** Verwendung nach Anspruch 11, **dadurch gekennzeichnet, dass** ein Teil der Zusammensetzung vorgehärtet wird, wonach erst der Rest der Zusammensetzung zugesetzt und das Gemisch ausgehärtet wird.

**13.** Verwendung nach Anspruch 11 oder 12, **dadurch gekennzeichnet, dass** die Polymerisation in einem generativen Fertigungsverfahren, z.B. Rapid-Prototyping- oder Rapid-Manufacturing-Verfahren, durchgeführt wird.

**14.** Verwendung nach einem der Ansprüche 11 bis 13, **dadurch gekennzeichnet, dass** das Polymer nach der Härtung einem oder mehreren Nachbehandlungsschritten unterzogen wird.

**15.** Verwendung nach Anspruch 14, **dadurch gekennzeichnet, dass** die Nachbehandlungsschritte aus Nachhärtungs-, Wärmebehandlungs-, Extraktions-, Umfällungs-, Sterilisations- und Oberflächenbehandlungsschritten ausgewählt werden.

## Claims

**1.** Use of a polymerization-curable composition for the preparation of biodegradable, biocompatible, cross-linked polymers, preferably on the basis of polyvinyl alcohol, for body implants and tissue support and regeneration material, said composition comprising:

a) 5 to 100 % by weight of one or more vinyl ester monomers which are independently selected from compounds of the general formulas (I) to (III):

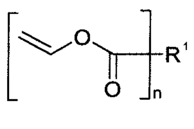
(I)

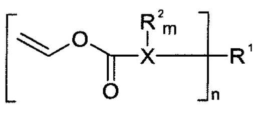
(II)

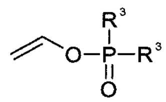
(III)

wherein:

X is a heteroatom selected from oxygen, sulfur, nitrogen, and phosphorus;
each n is independently 1 to 1000, preferably 1 to 50, more preferably 1 to 20, even more preferably 1 to 10, particularly 1 to 3, at least 20 % of the n being $\geq 2$;
each $R^1$ is independently selected from

i) hydrogen, linear, branched or cyclic, saturated or unsaturated, n-valent hydrocarbon residues having 1 to 30, preferably 3 to 25, even more preferably 4 to 20, particularly 5 to 15, carbon atoms, which optionally comprise one or more heteroatoms selected from oxygen, sulfur, nitrogen, and phosphorus within the chains and/or at the end of the chains, and which are optionally substituted with one or more substituents selected from -OH, -COOH, -CN, -CHO, and =O; and
ii) n-valent radicals of biodegradable, biocompatible oligomers and polymers, selected from polysaccharides, polypeptides, polyamides, polyesters, polycarbonates, polyethers, and fatty acid derivatives;

m is an integer from 0 to 4;
the groups $R^2$ are selected from hydrogen, -OH, =O, and the options for $R^1$; and
the groups $R^3$ are selected from hydrogen, -OH, and the options for $R^1$;

b) 0 to 50 % by weight of one or more ethylenically unsaturated co-monomers selected from (meth)acrylic acid, maleic acid, fumaric acid, vinylpyrrolidon and $\alpha$-olefin monomers;
c) 0 to 10 % by weight of one or more polymerization initiators selected from thermal initiators and photoinitiators; and
d) 0 to 95 % by weight of one or more solvents selected from water, lower alcohols, ether, ketone, ester, amide and hydrocarbon solvents;
the biodegradable, biocompatible, cross-linked polymers being produced by bringing the above-defined composition into a desired shape and thermally or photochemically polymerizing the same.

**2.** The use according to claim 1, **characterized in that** the at least one vinyl ester monomer of the general formula (I), (II) or (III) constitutes at least 50 mole % of all contained monomers.

**3.** The use according to claim 1 or 2, **characterized in that** at least 35, preferably at least 50, mole % of all vinyl ester monomers are bifunctional or higher functional monomers with $n \geq 2$, which act as cross-linkers.

**4.** The use according to any one of the claims 1 to 3, **characterized in that** the composition comprises at least one vinyl ester monomer of formula (III), wherein at least one of the groups $R^3$ is vinyloxy.

5. The use according to any one of the preceding claims, **characterized in that** at least one vinyl ester monomer is selected from 1,4-butanediol bis(vinyl carbonate), 2-cyanoethyl vinyl carbonate, N,N'-dimethyl-1,2-ethylenediamine bis(vinyl carbamate), sarcosine methyl ester vinyl carbamate, N,O-bis(vinyloxycarbonyl)-N-methyl-hydroxylamine, N-methoxy vinyl carbamate, N-acryloyl-N-methoxy vinyl carbamate, vinyloxycarbonylphosphonic acid diethyl ester, 2-(diethoxyphosphoryloxy)ethylamine vinyl carbamate, ethyl bis[2-(vinyloxycarbonylamino)ethyl] phosphate, and divinyl-ethyl phosphate.

6. The use according to any one of the preceding claims, **characterized in that** the co-monomers used as the component b) are selected from: (meth)acrylic anhydride, (meth)acrylic acid glycidyl ester, (meth)acryloyloxy succinic acid anhydride, (meth)acryloyloxymethyl succinic anhydride, (meth)acrylic acid 2-oxo-1,3-dioxolanylmethyl ester, vinyl succinic anhydride, vinylene carbonate, and maleic anhydride.

7. The use according to any one of the preceding claims, **characterized in that** it comprises, as a further component e), one or more additives selected from polymerization sensitizers and inhibitors, stabilizers, modifying agents, softeners, dyeing agents, bioactive agents, cells, e.g. osteoblasts, thickening agents, and fillers.

8. The use according to claim 7, **characterized in that** the bioactive agents are selected from drugs, proteins, antibodies, and ligands of cell surface receptors.

9. The use according to any one of the preceding claims, **characterized in that** one or more additives is/are covalently bound to monomers or co-monomers.

10. The use according to claim 9, **characterized in that** at least one additive covalently bound to monomers or co-monomers is a bioactive agent.

11. The use according to any one of the preceding claims, **characterized in that** a biodegradable, biocompatible, cross-linked polymer on the basis of polyvinyl alcohol is produced by thermal polymerization or photopolymerization of the composition.

12. The use according to claim 11, **characterized in that** part of the composition is pre-cured, whereafter the remaining composition is added and the mixture is cured.

13. The use according to claim 11 or claim 12, **characterized in that** the polymerization is carried out in the course of a generative manufacturing process such as a rapid prototyping or rapid manufacturing procedure.

14. The use according to any one of the claims 11 to 13, **characterized in that** the polymer is subjected to one or more post-treatment step(s) after curing.

15. The use according to claim 14, **characterized in that** the post-treatment steps are selected from post-curing, heat treatment, extraction, re-precipitation, sterilization, and surface treatment steps.

## Revendications

1. Utilisation d'une composition durcissable par polymérisation pour la production de polymères réticulés biodégradables et biocompatibles, de préférence à base d'alcool polyvinylique, pour des implants corporels et comme matière de support et de régénération du tissu, ladite composition comprenant:

a) 5 à 100 % en poids d'un ou plusieurs monomères d'ester vinylique indépendamment sélectionnés parmi les composés des formules générales (I) à (III):

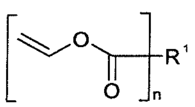

(I)

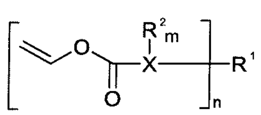

(II)

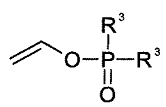

(III)

dans lesquelles
X est un hétéroatome sélectionné parmi l'oxygène, le soufre, l'azote et le phosphore;
les n sont indépendamment 1 à 1000, de préférence 1 à 50, plus particulièrement 1 à 20, plus particulièrement 1 à 20, plus particulièrement 1 à 10, notamment 1 à 3, 20 % des n étant $\geq 2$;
les $R^1$ sont indépendamment sélectionnés parmi

i) l'hydrogène, les radicaux d'hydrocarbures linéaires, ramifiés ou cycliques de valence n, ayant 1 à 30, de préférence de 3 à 25, plus particulièrement de 4 à 20, notamment de 5 à 15, atomes de carbone et, le cas échéant, avec un ou plusieurs hétéroatomes sélectionnés parmi l'oxygène, le soufre, l'azote et le phosphore à l'intérieur et/ou au bout de leur chaîne et, le cas échéant, substitués avec un ou plusieurs substituents sélectionnés parmi -OH, -COOH, -CN, -CHO et =O, et
ii) les radicaux de valence n d'oligomères et de polymères biodégradables et biocompatibles sélectionnés parmi les polysaccharides, polypeptides, polyamides, polyesters, polycarbonates, polyéthers et dérivées d'acides gras;

m est un intègre de 0 à 4;
les groupes $R^2$ sont sélectionnés parmi l'hydrogène, -OH, =O et les options pour $R^1$; et
les groupes $R^3$ sont sélectionnés parmi l'hydrogène, -OH, =O et les options pour $R^1$;
b) 0 à 50 % en poids d'un ou plusieurs co-monomère(s) non saturé(s) éthyléniquement sélectionné(s) parmi les monomères d'acide (meth)acrylique, maléique, fumarique, de vinyl pyrrolidone et d'alpha-oléfine;
c) 0 à 10 % en poids d'un ou plusieurs amorceur(s) de polymérisation sélectionné(s) des amorceurs thermiques et des photoamorceurs; et
d) 0 à 95 % en poids d'un ou plusieurs solvants sélectionné(s) parmi l'eau, les alcools de C1 à C5, des éthers, cétones, esters, amides et hydrocarbures;
les polymères réticulés biodégradables et biocompatibles étant produits en mettant en une forme désirée les compositions définis ci-dessus et en les soumettant à une polymérisation thermique ou photochimique.

**2.** Utilisation selon la revendication 1, **caractérisée en ce qu'**au moins un monomère d'ester vinylique selon l'une des formules générales (I), (II) ou (III) constitue au moins 50 % en moles de tous monomères.

**3.** Utilisation selon la revendication 1 ou 2, **caractérisée en ce qu'**au moins 35 % en moles, de préférence au moins 50 % en moles, de tous monomères d'ester vinylique sont de valence 2 ou plus et agissent comme agents de réticulation avec $n \geq 2$.

**4.** Utilisation selon l'une des revendications 1 à 3, **caractérisée en ce que** ladite composition comprend au moins un monomère d'ester vinylique de la formule (III), dans lequel au moins un des groupes $R^3$ est vinyloxy.

**5.** Utilisation selon l'une des revendications précédentes, **caractérisée en ce qu'**au moins un monomère d'ester vinylique est sélectionné parmi le 1,4-butanediolbis(vinylcarbonate), le 2-cyanoéthylvinylcarbonate, le N,N'-diméthyl-1,2-éthylènediamine-bis(vinylcarbamate), le vinylcarbamate d'ester méthylique de la sarcosine, le N,O-bis(vinyloxycarbonyl)-N-méthylhydroxylamine, le N-méthoxyvinylcarbamate, le N-acryloyl-N-méthoxyvinylcarbamate, l'ester diéthylique d'acide vinyloxycarbonylphosphonique, le 2-(diéthoxyphosphoryloxy)éthylamin-vinylcarbamate, l'éthyl-bis[2-(vinyloxycarbonylamino)éthyl]phosphate et le divinyléthylphosphate.

**6.** Utilisation selon l'une des revendications précédentes, **caractérisée en ce que** les co-monomères utilisés comme composant b) sont sélectionnés parmi: l'anhydride d'acide (meth)acrylique, l'ester glycidylique d'acide (meth)acrylique, l'anhydride d'acide (meth)acryloyloxysuccinique, l'anhydride d'acide (meth)acryloyloxyméthylsuccinique, l'ester 2-oxo-1,3-dioxolanméthylique d'acide (meth)acrylique, l'anhydride d'acide vinylsuccinique, le carbonate de vinylène et l'anhydride d'acide maléique.

**7.** Utilisation selon l'une des revendications précédentes, **caractérisée en ce que** ladite composition contient comme composant (e) un ou plusieurs additif(s) sélectionné(s) parmi les sensibilisateurs et inhibiteurs de polymérisation, les stabilisateurs, modificateurs, plastifiants, colorants, agents bioactifs, des cellules, comme par exemple les ostéoblastes, les épaississants et matières de charge.

**8.** Utilisation selon la revendication 7, **caractérisée en ce que** les agents bioactifs sont sélectionnés parmi les médicaments, protéines, anticorps et ligands des récepteurs des surfaces cellulaires.

9. Utilisation selon l'une des revendications précédentes, **caractérisée en ce qu'**un ou plusieurs additif(s) est/sont lié(s) de manière covalente à des monomères ou co-monomères.

10. Utilisation selon la revendication 9, **caractérisée en ce qu'**au moins un additif lié de manière covalente à des monomères ou co-monomères est un agent bioactif.

11. Utilisation selon l'une des revendications précédentes, **caractérisée en ce qu'**un polymère réticulé biodégradable et biocompatible à base de l'alcool polyvinylique est produit par la polymérisation thermique ou la photopolymérisation de ladite composition.

12. Utilisation selon la revendication 11, **caractérisée en ce qu'**une partie de la composition est pré-durcie, le reste de la composition étant ensuite ajouté et le mélange étant durci.

13. Utilisation selon la revendication 11 ou 12, **caractérisée en ce que** la polymérisation fait partie d'un procédé de fabrication additif, comme par exemple d'un procédé de prototypage rapide ou de fabrication rapide.

14. Utilisation selon l'une des revendications 11 à 13, **caractérisée en ce que** le polymère est soumis à une ou plusieurs étapes de post-traitement après le durcissement.

15. Utilisation selon la revendication 14, **caractérisée en ce que** les étapes de traitement postérieur sont choisies parmi les étapes de post-durcissement, de traitement thermique, d'extraction, de ré-précipitation, de stérilisation et de traitement de surface.

FIGUR 1

Fig. 1a
Acrylat

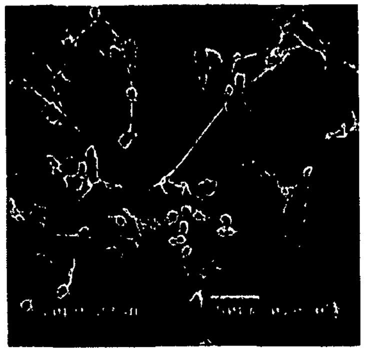

Fig. 1b
Vinylester

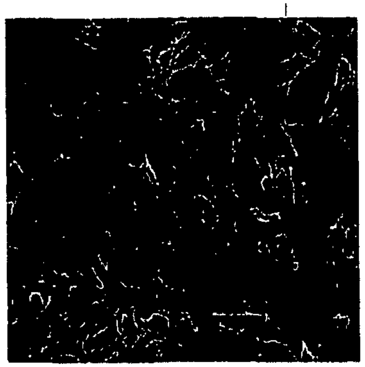

Fig. 1c
Vinylester

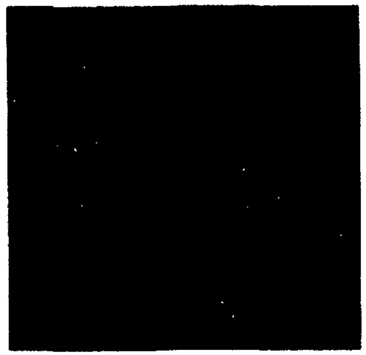

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20040110439 A
- WO 2006108202 A
- JP 09143194 A
- JP 2000086806 A
- JP 2003321624 A
- JP 2001316465 A
- JP 11276188 A
- WO 9318070 A1
- EP 629214 B1
- WO 200337944 A
- WO 200671388 A
- WO 200671479 A
- WO 200174932 A


### In der Beschreibung aufgeführte Nicht-Patentliteratur


- **T. MATSUDA ; M. MIZUTANI ; S. ARNOLD.** *Macromolecules,* 2000, vol. 33, 795-800
- **M. MIZUTANI ; T. MATSUDA.** *Journal of Biomedical Materials Research,* 2002, vol. 61 (1), 53-60
- **M. MIZUTANI ; T. MATSUDA.** *Journal of Biomedical Materials Research,* 2002, vol. 62, 395
- **L. XUE ; H.P. GREISLER.** Biomaterials in the development and future of vascular grafts. *J. Vasc. Surg.,* 2003, vol. 37, 472-480
- **R.A. OLOFSON ; J. CUOMO.** *Tetrahedron Lett.,* 1980, vol. 21 (9), 819-22
- **R.A. OLOFSON ; DANG VU ANH ; D.S. MORRISON ; P.F. DE CUSATI.** *J. Org. Chem.,* 1990, vol. 55 (1), 1-3
- **K. REGE ; S. HU ; J.A. MOORE ; J.S. DORDICK ; S.M. CRAMER.** *J. Am. Chem. Soc.,* 2004, vol. 126 (39), 12306-12315
- **R.A. OLOFSON ; B.A. BAUMAN ; D.J. WANCOWICZ.** *J. Org. Chem.,* 1978, vol. 43 (4), 752-4
- **A.J. DUGGAN ; F.E. ROBERTS.** *Tetrahedron Lett.,* 1979, vol. 20 (7), 595-8
- **R.A. OLOFSON ; J. CUOMO.** *J. Org. Chem.,* 1980, vol. 45 (12), 2538-41
- **M.I. WEINHOUSE ; K.D. JANDA.** A new methodology for the preparation of vinyl esters. *Synthesis,* 1993, vol. 1, 81-83
- **D.C. WEBSTER.** Cyclic carbonate functional polymers and their applications. *Progress in Organic Coatings,* 2003, vol. 47 (1), 77-86
- **W.C. OLIVER ; G.M. PHARR.** *J. Mater. Res.,* 1992, vol. 7, 1564